# EUROPEAN PATENT APPLICATION

(11) **EP 2 277 534 A1**
(43) Date of publication of application: **26.01.2011**
(21) Application number: 09165927.6
(22) Date of filing: 20.07.2009
(51) Int. Cl.: A61K 39/09, C07K 14/315, G01N 33/569

(54) **Immunogenic and therapeutic compositions for streptococcus suis**

(71) Applicant: Universidad De Córdoba, 14071 Córdoba (ES); Beninati, Concetta, 98125 Messina (IT)
(72) Inventor: Beninati, Concetta, 98167 Messina (IT); Rodríguez Ortega, Manuel José, 14071 Córdoba (ES)
(74) Representative: Spadaro, Marco

(57) **Abstract**

The present invention refers to protein Ssu05_0473 and protein Ssu05_0272 or immunogenic derivatives thereof for use as a medicament, in particular for use as a medicament against Streptococcus suis colonization or infection in humans and animals. The present invention also refers to a vaccine comprising at least one of said proteins or immunogenic derivatives thereof, to a method for the diagnosis of bacterial infections using said proteins or immunogenic derivatives thereof and to a kit for the diagnosis of Streptococcus suis infections.

SEQUENCE LISTING

<110> BENINATI, CONCETTA
      UNIVERSIDAD DE CORDOBA

<120> IMMUNOGENIC AND THERAPEUTIC COMPOSITIONS FOR STREPTOCOCCUS
      SUIS

<130> 09105MS

<140>
<141>

<160> 17

<170> PatentIn version 3.5

<210> 1
<211> 5
<212> PRT
<213> Artificial Sequence

<220>
<221> source
<223> /note="Description of Artificial Sequence: Synthetic
      peptide"

<220>
<221> MOD_RES
<222> (3)..(3)
<223> Any amino acid

<400> 1
Leu Pro Xaa Thr Gly
1               5

<210> 2
<211> 4812
<212> DNA
<213> Streptococcus suis

<400> 2
gtgcaagaaa caccagtaac aacggaaatt agtaaattaa cagaaccgat ggtgacaact      60

cagcaaactc cgatagtggg tagctctgct gatgaagttc aatctcctgc gacagaaacg     120

gttcctgacc ctgctgagca agtgtcaaca gagtctgcta gcaaacccca gaccgaccaa     180

gaacggatta ctgagccaac taccatagtt cacaaaacga atgattatga ggtgactgca     240

acatttgatg cgagtgctca atttcccaaa gatgttgagt ttaatgttag agaactagat     300

agccaatcag aagaatatcg gacacattat gaaaaaacca aggagaagct tggagtaaaa     360

aaacttgtat atgcccgatt ctttgatatt catttttcat cacagggcca agaggtcgag     420

cctgcctccc ctgtgaagat tacgattaga aacaaggaat tagttcaacg gccagatgat     480

agtaaactga aaattgtcca ctttgaagag aatagccaga tagaagtttt agaggccgaa     540

acgaaggaaa atgaacaaca aatctccgag atcagttttg acgctagcca tttttctgtt     600

tatggagatg ttttagcaaa ctattatact gtcaaattta cctacactga cacagcaggt     660

caagaacaag tcattacgag tttgattgat aagactgtgg ggacttcact tggtagtttg     720

cctgaagctc cttttaaaaa cggttatgta ttttctcatt ggattaataa agatactggt     780

gatcggattg atgaaaatac ggtgataact ggcgacctga ctgctgtagc agtatttaat 840
aatatcaata tctacaccat tgatgtcacg tatttctacc acaatgtttc taaaaaccaa 900
gatattacga ttgataagga aacggtgcaa ctggaagcaa gtgatgctcc atatcaactg 960
acaccacctg cttctactga ggtgagcaac aaagaggata caactttacc agcagatgct 1020
atttactacc ctgaaaaacc acttcttgag tttacagcag atcaactaga gcgactggac 1080
gtagcagatg gagtggatga taattatatt agcgaaagag tacaatatgt tccttacaat 1140
gcagagtatg atgtcattta taggctaaag aatttgacgg ggtcaggtta ttctgatatc 1200
cagactgttc atgtccgtgg ggtactgggt tcaactgtta caccgcaaat tctaacctac 1260
aactatgctg actttgaacg tgctgataca gcaaagattg agaatactag cggccaaaaa 1320
ttgtatgttt attatacacg gaaaaccttt actctttctt ataattccaa tggcggtagt 1380
aatattcccc aacaacaggg tctatacgat agccagatta agatttctac aactactcca 1440
acaaaagagg ggtatacatt tgaaggttgg tatgataatc ctggactgac tggaaacaag 1500
attacaggct ctctcacctt aaagaaagat acaactttgt atgcgaaatg gacaccgaac 1560
agggttcgct acatcattgg gtattacaag gaagtctatg acaacaacac tggaacaacc 1620
tcctatgtat atgatagttc ggcagctggt tggggtctgg ttgggactac tgtaaaagga 1680
acagattcaa atattggacc tcctccgcca acccaaacaa ccattggcag cattggctac 1740
gagaaggatg ttactaaaag tgctgcttca agtattgaaa ttgcagctga tggaacatct 1800
gttttgaaag tttactataa actgaagcgc tacacttttg aatttttctt aaatgataac 1860
aatgccaata tcaccataaa tggtaagacc tactctgata ggaataggta catgatacgt 1920
gatgttgtgc ttggtcaaga tgtctcaaag ttatggccag cttctataac aaatcctaaa 1980
gaagtatacg atgataaagt ctactatgat agctatggta ggccgataag atactttttt 2040
gactactgga tttttgggaa tctactcttc aaaacagatc ggttcgaagt tgttgagtct 2100
atgttgccaa cgagtggaac aaccagtaag ttcacagcat attggaatac ccaaggtaca 2160
caggctaacg ttgaatattg gttacaacaa ccggatggtt cttataagaa gtctgatata 2220
tatagtcaat ctttcctaaa gactgcagga ctgctaccga aagaaatttt tggatattct 2280
tattatacag atgctagttt tactgaagca ggcattaggt attacggaac tcaaggaaat 2340
acatatcgtt tctactataa acgccaagcc ttcaaaattg actattatta caatagtact 2400
gggttagatt caaagagtaa cattctcttt gagagtaaca tcaatacagc aacttataat 2460
taccaaccac cacgttctac tggagttgat gctgattata catggggtgg atggtataca 2520
gattcgttat tgacagagaa gtataacttt aataagatgc ctgccaataa cttggttctc 2580
tatgctaagt ggattgctcc aaaatttaag gttacttttg atatcaacgg tggtaatgga 2640
actgcccctg tcgatcaaag tgttgaaaaa tacaaatacg ctaaggcccc tgctgatcca 2700
acgagggaac attacaattt ccttggttgg tctacagaag atgggaaact ctatagttgg 2760
agcgaccaag taaccaagga tgtcaaattg actgcgaagt gggaattgaa accattaact 2820

tatactgttc gctatcttga agcaggtaca aatacgcaat tggctgctga taaagtgatt 2880
acgagtccag cgcttgtatt gaatcaagct atcacagaaa aggcattggg aattacaggt 2940
tatcgaccag ataagagtag tcaaacaatt aacctgaatt ttagagggaa cgaaataact 3000
ttctactata caccgaaggt aaaagatgtt gtttacacca tcaagtatgt cttgaaggat 3060
aacccagcta ttgaggtagc acctacagtt actcgtaagg ttgatggaag tatcatccgt 3120
gcgaaagaag ctgctgcaga tgtgaataaa cagcatttgc agagtcagtc tggtgttact 3180
gctgaaatgc tggaaatgga ttactatcca actgtaaata ctcaaagttt gattctaact 3240
tctagtgatg caaataatgt gattgttttt gaatatgtcg gattcgatac tcaaaccttt 3300
acggtaaatt atttggatat ggatggtaag aaaatcgatg gtcaagaacc attggtagtt 3360
taccgaaaga aaccaggtag tttcgtggta gatcataaga ctataaatgg ttatacctat 3420
gatcattcgt tagacagtga gaatgcagct gataagacag tataccgtgt aagtaagggt 3480
ggtcacataa ccattgatct ctattataag aaaaacctaa cactgaccgc attagataag 3540
tccaaagttt atgatggcac tgccctatca agttcgggtc tgtctgattt aaatccttcg 3600
tacagttcag aactgaaaac aggtgacaaa ttggttgcca tcagctatga tggaagtcaa 3660
acaaatgctg gtagtagcgc aacgacacct aaacttgcta acatcagcaa tgcatctggt 3720
gctgaccgga cgaatttcta tcacataacc tatcaaccgg gtaacttaag agttgaccaa 3780
cagccagttg tggttgtcat cgacggtgaa aagaaatcga aaatctatga tggcaaagca 3840
gaagcagttg gttataaggt gacggaaata agagattcaa gcggtctcta taaaaaaaca 3900
gatatcaaat ttaatggttc aagttcagag cagtcggtta agaaaaaaga tgcaggaatt 3960
tatccactcc ttttaagtaa tagattctca aataccaatc agaatttcca agtagatttc 4020
ttgatttctg atggggaatt aagaatagat agacggcaac tgaccataac atctgactcc 4080
gctagcaagg cttatgacgg tagcgagctt cgtgctgaca atatcacagt ctctgtacct 4140
gctggtgttg actacacagg ttttgtcgat ggcgaaggga ttataccaac cttcttaggc 4200
ggtccaatcg attctggaat gatgccaaac ttctttgaat atgctctaaa atctggcacg 4260
aatttagcta attatgagtt agatatccag tttggccagc tcaaggtcaa agaagtcatc 4320
aagattcaga aaacagacct ggcctggcag gccctttcag gtggtaagtt tgaattgacc 4380
aagtgggatg gtttgaactg ggctcaggta gatggggtgc aggaatttgc cattaccagc 4440
aaagatggta ttcggatacc ggtaggctta gaagcaggtc gctaccgtct gcaagaattg 4500
gcagcacctg atggttttat cgtcttggat agctatatct acttcagcat caaggaaatc 4560
tttaacgaag ataagacatc ttccttctac acagtttcct tgtctgatga agcaggcaag 4620
gatgctagtc cggaacgagc aacgcttacc aaggttagtg gtgatgccag tcatcgcatc 4680
caagtggcca atgagcaggg cagagccttg ccaagcacag gcggagatgg acagaagtgg 4740
tttattcttt ctggccttgt cctcatagcc atctcctatc tgatgcatct gtttgttcaa 4800
cgtcgaaggt aa 4812

<210> 3
<211> 1603
<212> PRT
<213> Streptococcus suis

<400> 3
Met Gln Glu Thr Pro Val Thr Thr Glu Ile Ser Lys Leu Thr Glu Pro
1               5                   10                  15

Met Val Thr Thr Gln Gln Thr Pro Ile Val Gly Ser Ser Ala Asp Glu
            20                  25                  30

Val Gln Ser Pro Ala Thr Glu Thr Val Pro Asp Pro Ala Glu Gln Val
        35                  40                  45

Ser Thr Glu Ser Ala Ser Lys Pro Gln Thr Asp Gln Glu Arg Ile Thr
    50                  55                  60

Glu Pro Thr Thr Ile Val His Lys Thr Asn Asp Tyr Glu Val Thr Ala
65                  70                  75                  80

Thr Phe Asp Ala Ser Ala Gln Phe Pro Lys Asp Val Glu Phe Asn Val
                85                  90                  95

Arg Glu Leu Asp Ser Gln Ser Glu Glu Tyr Arg Thr His Tyr Glu Lys
            100                 105                 110

Thr Lys Glu Lys Leu Gly Val Lys Lys Leu Val Tyr Ala Arg Phe Phe
        115                 120                 125

Asp Ile His Phe Ser Ser Gln Gly Gln Glu Val Glu Pro Ala Ser Pro
    130                 135                 140

Val Lys Ile Thr Ile Arg Asn Lys Glu Leu Val Gln Arg Pro Asp Asp
145                 150                 155                 160

Ser Lys Leu Lys Ile Val His Phe Glu Glu Asn Ser Gln Ile Glu Val
                165                 170                 175

Leu Glu Ala Glu Thr Lys Glu Asn Glu Gln Gln Ile Ser Glu Ile Ser
            180                 185                 190

Phe Asp Ala Ser His Phe Ser Val Tyr Gly Asp Val Leu Ala Asn Tyr
        195                 200                 205

Tyr Thr Val Lys Phe Thr Tyr Thr Asp Thr Ala Gly Gln Glu Gln Val
    210                 215                 220

Ile Thr Ser Leu Ile Asp Lys Thr Val Gly Thr Ser Leu Gly Ser Leu
225                 230                 235                 240

Pro Glu Ala Pro Phe Lys Asn Gly Tyr Val Phe Ser His Trp Ile Asn
                245                 250                 255

Lys Asp Thr Gly Asp Arg Ile Asp Glu Asn Thr Val Ile Thr Gly Asp
            260                 265                 270

Leu Thr Ala Val Ala Val Phe Asn Asn Ile Asn Ile Tyr Thr Ile Asp
        275                 280                 285

Val Thr Tyr Phe Tyr His Asn Val Ser Lys Asn Gln Asp Ile Thr Ile
    290                 295                 300

Asp Lys Glu Thr Val Gln Leu Glu Ala Ser Asp Ala Pro Tyr Gln Leu
305                 310                 315                 320

Thr Pro Pro Ala Ser Thr Glu Val Ser Asn Lys Glu Asp Thr Thr Leu
                325                 330                 335

Pro Ala Asp Ala Ile Tyr Tyr Pro Glu Lys Pro Leu Leu Glu Phe Thr
            340                 345                 350

Ala Asp Gln Leu Glu Arg Leu Asp Val Ala Asp Gly Val Asp Asp Asn
        355                 360                 365

Tyr Ile Ser Glu Arg Val Gln Tyr Val Pro Tyr Asn Ala Glu Tyr Asp
    370                 375                 380

Val Ile Tyr Arg Leu Lys Asn Leu Thr Gly Ser Gly Tyr Ser Asp Ile
385                 390                 395                 400

Gln Thr Val His Val Arg Gly Val Leu Gly Ser Thr Val Thr Pro Gln
                405                 410                 415

Ile Leu Thr Tyr Asn Tyr Ala Asp Phe Glu Arg Ala Asp Thr Ala Lys
            420                 425                 430

Ile Glu Asn Thr Ser Gly Gln Lys Leu Tyr Val Tyr Tyr Thr Arg Lys
        435                 440                 445

Thr Phe Thr Leu Ser Tyr Asn Ser Asn Gly Gly Ser Asn Ile Pro Gln
    450                 455                 460

Gln Gln Gly Leu Tyr Asp Ser Gln Ile Lys Ile Ser Thr Thr Thr Pro
465                 470                 475                 480

Thr Lys Glu Gly Tyr Thr Phe Glu Gly Trp Tyr Asp Asn Pro Gly Leu
                485                 490                 495

Thr Gly Asn Lys Ile Thr Gly Ser Leu Thr Leu Lys Lys Asp Thr Thr
            500                 505                 510

Leu Tyr Ala Lys Trp Thr Pro Asn Arg Val Arg Tyr Ile Ile Gly Tyr
        515                 520                 525

Tyr Lys Glu Val Tyr Asp Asn Asn Thr Gly Thr Thr Ser Tyr Val Tyr
    530                 535                 540

Asp Ser Ser Ala Ala Gly Trp Gly Leu Val Gly Thr Thr Val Lys Gly
545                 550                 555                 560

Thr Asp Ser Asn Ile Gly Pro Pro Pro Pro Thr Gln Thr Thr Ile Gly
                565                 570                 575

Ser Ile Gly Tyr Glu Lys Asp Val Thr Lys Ser Ala Ala Ser Ser Ile
            580                 585                 590

Glu Ile Ala Ala Asp Gly Thr Ser Val Leu Lys Val Tyr Tyr Lys Leu
        595                 600                 605

Lys Arg Tyr Thr Phe Glu Phe Phe Leu Asn Asp Asn Asn Ala Asn Ile
    610                 615                 620

Thr Ile Asn Gly Lys Thr Tyr Ser Asp Arg Asn Arg Tyr Met Ile Arg
625                 630                 635                 640

Asp Val Val Leu Gly Gln Asp Val Ser Lys Leu Trp Pro Ala Ser Ile
                645                 650                 655

Thr Asn Pro Lys Glu Val Tyr Asp Asp Lys Val Tyr Tyr Asp Ser Tyr
            660                 665                 670

Gly Arg Pro Ile Arg Tyr Phe Phe Asp Tyr Trp Ile Phe Gly Asn Leu
        675                 680                 685

Leu Phe Lys Thr Asp Arg Phe Glu Val Val Glu Ser Met Leu Pro Thr
    690                 695                 700

Ser Gly Thr Thr Ser Lys Phe Thr Ala Tyr Trp Asn Thr Gln Gly Thr
705                 710                 715                 720

Gln Ala Asn Val Glu Tyr Trp Leu Gln Gln Pro Asp Gly Ser Tyr Lys
                725                 730                 735

Lys Ser Asp Ile Tyr Ser Gln Ser Phe Leu Lys Thr Ala Gly Leu Leu
            740                 745                 750

Pro Lys Glu Ile Phe Gly Tyr Ser Tyr Tyr Thr Asp Ala Ser Phe Thr
        755                 760                 765

Glu Ala Gly Ile Arg Tyr Tyr Gly Thr Gln Gly Asn Thr Tyr Arg Phe
    770                 775                 780

Tyr Tyr Lys Arg Gln Ala Phe Lys Ile Asp Tyr Tyr Tyr Asn Ser Thr
785                 790                 795                 800

Gly Leu Asp Ser Lys Ser Asn Ile Leu Phe Glu Ser Asn Ile Asn Thr

                805                 810                 815

Ala Thr Tyr Asn Tyr Gln Pro Pro Arg Ser Thr Gly Val Asp Ala Asp
            820                 825                 830

Tyr Thr Trp Gly Gly Trp Tyr Thr Asp Ser Leu Leu Thr Glu Lys Tyr
        835                 840                 845

Asn Phe Asn Lys Met Pro Ala Asn Asn Leu Val Leu Tyr Ala Lys Trp
    850                 855                 860

Ile Ala Pro Lys Phe Lys Val Thr Phe Asp Ile Asn Gly Gly Asn Gly
865                 870                 875                 880

Thr Ala Pro Val Asp Gln Ser Val Glu Lys Tyr Lys Tyr Ala Lys Ala
                885                 890                 895

Pro Ala Asp Pro Thr Arg Glu His Tyr Asn Phe Leu Gly Trp Ser Thr
            900                 905                 910

Glu Asp Gly Lys Leu Tyr Ser Trp Ser Asp Gln Val Thr Lys Asp Val
        915                 920                 925

Lys Leu Thr Ala Lys Trp Glu Leu Lys Pro Leu Thr Tyr Thr Val Arg
    930                 935                 940

Tyr Leu Glu Ala Gly Thr Asn Thr Gln Leu Ala Ala Asp Lys Val Ile
945                 950                 955                 960

Thr Ser Pro Ala Leu Val Leu Asn Gln Ala Ile Thr Glu Lys Ala Leu
                965                 970                 975

Gly Ile Thr Gly Tyr Arg Pro Asp Lys Ser Ser Gln Thr Ile Asn Leu
            980                 985                 990

Asn Phe Arg Gly Asn Glu Ile Thr Phe Tyr Tyr Thr Pro Lys Val Lys
        995                 1000                1005

Asp Val Val Tyr Thr Ile Lys Tyr Val Leu Lys Asp Asn Pro Ala
    1010                1015                1020

Ile Glu Val Ala Pro Thr Val Thr Arg Lys Val Asp Gly Ser Ile
    1025                1030                1035

Ile Arg Ala Lys Glu Ala Ala Ala Asp Val Asn Lys Gln His Leu
    1040                1045                1050

Gln Ser Gln Ser Gly Val Thr Ala Glu Met Leu Glu Met Asp Tyr
    1055                1060                1065

Tyr Pro Thr Val Asn Thr Gln Ser Leu Ile Leu Thr Ser Ser Asp
    1070                1075                1080

Ala Asn Asn Val Ile Val Phe Glu Tyr Val Gly Phe Asp Thr Gln
    1085                1090                1095

Thr Phe Thr Val Asn Tyr Leu Asp Met Asp Gly Lys Lys Ile Asp
    1100                1105                1110

Gly Gln Glu Pro Leu Val Val Tyr Arg Lys Lys Pro Gly Ser Phe
    1115                1120                1125

Val Val Asp His Lys Thr Ile Asn Gly Tyr Thr Tyr Asp His Ser
    1130                1135                1140

Leu Asp Ser Glu Asn Ala Ala Asp Lys Thr Val Tyr Arg Val Ser
    1145                1150                1155

Lys Gly Gly His Ile Thr Ile Asp Leu Tyr Tyr Lys Lys Asn Leu
    1160                1165                1170

Thr Leu Thr Ala Leu Asp Lys Ser Lys Val Tyr Asp Gly Thr Ala
    1175                1180                1185

Leu Ser Ser Ser Gly Leu Ser Asp Leu Asn Pro Ser Tyr Ser Ser
    1190                1195                1200

Glu Leu Lys Thr Gly Asp Lys Leu Val Ala Ile Ser Tyr Asp Gly
    1205                1210                1215

Ser Gln Thr Asn Ala Gly Ser Ser Ala Thr Thr Pro Lys Leu Ala
    1220                1225                1230

Asn Ile Ser Asn Ala Ser Gly Ala Asp Arg Thr Asn Phe Tyr His
    1235                1240                1245

Ile Thr Tyr Gln Pro Gly Asn Leu Arg Val Asp Gln Gln Pro Val
    1250                1255                1260

Val Val Val Ile Asp Gly Glu Lys Lys Ser Lys Ile Tyr Asp Gly
    1265                1270                1275

Lys Ala Glu Ala Val Gly Tyr Lys Val Thr Glu Ile Arg Asp Ser
    1280                1285                1290

Ser Gly Leu Tyr Lys Lys Thr Asp Ile Lys Phe Asn Gly Ser Ser
    1295                1300                1305

Ser Glu Gln Ser Val Lys Lys Lys Asp Ala Gly Ile Tyr Pro Leu
    1310                1315                1320

Leu Leu Ser Asn Arg Phe Ser Asn Thr Asn Gln Asn Phe Gln Val
    1325                1330                1335

Asp Phe Leu Ile Ser Asp Gly Glu Leu Arg Ile Asp Arg Arg Gln
    1340                1345                1350

Leu Thr Ile Thr Ser Asp Ser Ala Ser Lys Ala Tyr Asp Gly Ser
    1355                1360                1365

Glu Leu Arg Ala Asp Asn Ile Thr Val Ser Val Pro Ala Gly Val
    1370                1375                1380

Asp Tyr Thr Gly Phe Val Asp Gly Glu Gly Ile Ile Pro Thr Phe
    1385                1390                1395

Leu Gly Gly Pro Ile Asp Ser Gly Met Met Pro Asn Phe Phe Glu
    1400                1405                1410

Tyr Ala Leu Lys Ser Gly Thr Asn Leu Ala Asn Tyr Glu Leu Asp
    1415                1420                1425

Ile Gln Phe Gly Gln Leu Lys Val Lys Glu Val Ile Lys Ile Gln
    1430                1435                1440

Lys Thr Asp Leu Ala Trp Gln Ala Leu Ser Gly Gly Lys Phe Glu
    1445                1450                1455

Leu Thr Lys Trp Asp Gly Leu Asn Trp Ala Gln Val Asp Gly Val
    1460                1465                1470

Gln Glu Phe Ala Ile Thr Ser Lys Asp Gly Ile Arg Ile Pro Val
    1475                1480                1485

Gly Leu Glu Ala Gly Arg Tyr Arg Leu Gln Glu Leu Ala Ala Pro
    1490                1495                1500

Asp Gly Phe Ile Val Leu Asp Ser Tyr Ile Tyr Phe Ser Ile Lys
    1505                1510                1515

Glu Ile Phe Asn Glu Asp Lys Thr Ser Ser Phe Tyr Thr Val Ser
    1520                1525                1530

Leu Ser Asp Glu Ala Gly Lys Asp Ala Ser Pro Glu Arg Ala Thr
    1535                1540                1545

Leu Thr Lys Val Ser Gly Asp Ala Ser His Arg Ile Gln Val Ala
    1550                1555                1560

Asn Glu Gln Gly Arg Ala Leu Pro Ser Thr Gly Gly Asp Gly Gln
    1565                1570                1575

Lys Trp Phe Ile Leu Ser Gly Leu Val Leu Ile Ala Ile Ser Tyr
    1580                1585                1590

Leu Met His Leu Phe Val Gln Arg Arg Arg
    1595                1600

<210> 4
<211> 1217
<212> DNA
<213> Artificial Sequence

<220>
<221> source
<223> /note="Description of Artificial Sequence: Synthetic
      polynucleotide"

<400> 4
ggatccggga gtctgctagc aaaccccaga ccgaccaaga acggattact gagccaacta      60

ccatagttca caaaacgaat gattatgagg tgactgcaac atttgatgcg agtgctcaat     120

ttcccaaaga tgttgagttt aatgttagag aactagatag ccaatcagaa gaatatcgga     180

cacattatga aaaaaccaag gagaagcttg gagtaaaaaa acttgtatat gcccgattct     240

ttgatattca tttttcatca cagggccaag aggtcgagcc tgcctcccct gtgaagatta     300

cgattagaaa caaggaatta gttcaacggc cagatgatag taaactgaaa attgtccact     360

ttgaagagaa tagccagata gaagttttag aggccgaaac gaaggaaaat gaacaacaaa     420

tctccgagat cagttttgac gctagccatt tttctgttta tggagatgtt ttagcaaact     480

attatactgt caaatttacc tacactgaca cagcaggtca agaacaagtc attacgagtt     540

tgattgataa gactgtgggg acttcacttg gtagtttgcc tgaagctcct tttaaaaacg     600

gttatgtatt ttctcattgg attaataaag atactggtga tcggattgat gaaaatacgg     660

tgataactgg cgacctgact gctgtagcag tatttaataa tatcaatatc tacaccattg     720

atgtcacgta tttctaccac aatgtttcta aaaaccaaga tattacgatt gataaggaaa     780

cggtgcaact ggaagcaagt gatgctccat atcaactgac accacctgct tctactgagg     840

tgagcaacaa agaggataca actttaccag cagatgctat ttactaccct gaaaaaccac     900

ttcttgagtt tacagcagat caactagagc gactggacgt agcagatgga gtggatgata     960

attatattag cgaaagagta caatatgttc cttacaatgc agagtatgat gtcatttata    1020

ggctaaagaa tttgacgggg tcaggttatt ctgatatcca gactgttcat gtccgtgggg    1080

tactgggttc aactgttaca ccgcaaattc taacctacaa ctatgctgac tttgaacgtg    1140

ctgatacagc aaagattgag aatactagcg gccaaaaatt gtatgtttat tatacacgga    1200

aaacctttgg cggccgc                                                   1217

<210> 5
<211> 544
<212> PRT
<213> Artificial Sequence

<220>
<221> source
<223> /note="Description of Artificial Sequence: Synthetic
      polypeptide"

<400> 5

Met Lys Leu Lys Val Thr Val Asn Gly Thr Ala Tyr Asp Val Asp Val
1               5                   10                  15

Asp Val Asp Lys Ser His Glu Asn Pro Met Gly Thr Ile Leu Phe Gly
            20                  25                  30

Gly Gly Thr Gly Gly Ala Pro Ala Pro Ala Ala Gly Gly Ala Gly Ala
        35                  40                  45

Gly Lys Ala Gly Glu Gly Glu Ile Pro Ala Pro Leu Ala Gly Thr Val
    50                  55                  60

Ser Lys Ile Leu Val Lys Glu Gly Asp Thr Val Lys Ala Gly Gln Thr
65                  70                  75                  80

Val Leu Val Leu Glu Ala Met Lys Met Glu Thr Glu Ile Asn Ala Pro
                85                  90                  95

Thr Asp Gly Lys Val Glu Lys Val Leu Val Lys Glu Arg Asp Ala Val
            100                 105                 110

Gln Gly Gly Gln Gly Leu Ile Lys Ile Gly Asp Leu Glu Leu Ile Glu
        115                 120                 125

Gly Arg Glu Ala Ser Ala Gly Ile Arg Glu Ser Ala Ser Lys Pro Gln
    130                 135                 140

Thr Asp Gln Glu Arg Ile Thr Glu Pro Thr Thr Ile Val His Lys Thr
145                 150                 155                 160

Asn Asp Tyr Glu Val Thr Ala Thr Phe Asp Ala Ser Ala Gln Phe Pro
                165                 170                 175

Lys Asp Val Glu Phe Asn Val Arg Glu Leu Asp Ser Gln Ser Glu Glu
            180                 185                 190

Tyr Arg Thr His Tyr Glu Lys Thr Lys Glu Lys Leu Gly Val Lys Lys
        195                 200                 205

Leu Val Tyr Ala Arg Phe Phe Asp Ile His Phe Ser Ser Gln Gly Gln
    210                 215                 220

Glu Val Glu Pro Ala Ser Pro Val Lys Ile Thr Ile Arg Asn Lys Glu
225                 230                 235                 240

Leu Val Gln Arg Pro Asp Asp Ser Lys Leu Lys Ile Val His Phe Glu
                245                 250                 255

Glu Asn Ser Gln Ile Glu Val Leu Glu Ala Glu Thr Lys Glu Asn Glu
            260                 265                 270

Gln Gln Ile Ser Glu Ile Ser Phe Asp Ala Ser His Phe Ser Val Tyr

        275                 280                 285

Gly Asp Val Leu Ala Asn Tyr Tyr Thr Val Lys Phe Thr Tyr Thr Asp
    290                 295                 300

Thr Ala Gly Gln Glu Gln Val Ile Thr Ser Leu Ile Asp Lys Thr Val
305                 310                 315                 320

Gly Thr Ser Leu Gly Ser Leu Pro Glu Ala Pro Phe Lys Asn Gly Tyr
                325                 330                 335

Val Phe Ser His Trp Ile Asn Lys Asp Thr Gly Asp Arg Ile Asp Glu
            340                 345                 350

Asn Thr Val Ile Thr Gly Asp Leu Thr Ala Val Ala Val Phe Asn Asn
        355                 360                 365

Ile Asn Ile Tyr Thr Ile Asp Val Thr Tyr Phe Tyr His Asn Val Ser
    370                 375                 380

Lys Asn Gln Asp Ile Thr Ile Asp Lys Glu Thr Val Gln Leu Glu Ala
385                 390                 395                 400

Ser Asp Ala Pro Tyr Gln Leu Thr Pro Pro Ala Ser Thr Glu Val Ser
                405                 410                 415

Asn Lys Glu Asp Thr Thr Leu Pro Ala Asp Ala Ile Tyr Tyr Pro Glu
            420                 425                 430

Lys Pro Leu Leu Glu Phe Thr Ala Asp Gln Leu Glu Arg Leu Asp Val
        435                 440                 445

Ala Asp Gly Val Asp Asp Asn Tyr Ile Ser Glu Arg Val Gln Tyr Val
    450                 455                 460

Pro Tyr Asn Ala Glu Tyr Asp Val Ile Tyr Arg Leu Lys Asn Leu Thr
465                 470                 475                 480

Gly Ser Gly Tyr Ser Asp Ile Gln Thr Val His Val Arg Gly Val Leu
                485                 490                 495

Gly Ser Thr Val Thr Pro Gln Ile Leu Thr Tyr Asn Tyr Ala Asp Phe
            500                 505                 510

Glu Arg Ala Asp Thr Ala Lys Ile Glu Asn Thr Ser Gly Gln Lys Leu
        515                 520                 525

Tyr Val Tyr Tyr Thr Arg Lys Thr Phe Gly Gly Arg Asp Leu Val Leu
    530                 535                 540

<210> 6
<211> 1217

<212> DNA
<213> Artificial Sequence

<220>
<221> source
<223> /note="Description of Artificial Sequence: Synthetic
      polynucleotide"

<400> 6
ggatccggaa taagatgcct gccaataact tggttctcta tgctaagtgg attgctccaa      60

aatttaaggt tacttttgat atcaacggtg gtaatggaac tgcccctgtc gatcaaagtg     120

ttgaaaaata caaatacgct aaggcccctg ctgatccaac gagggaacat tacaatttcc     180

ttggttggtc tacagaagat gggaaactct atagttggag cgaccaagta accaaggatg     240

tcaaattgac tgcgaagtgg gaattgaaac cattaactta tactgttcgc tatcttgaag     300

caggtacaaa tacgcaattg gctgctgata aagtgattac gagtccagcg cttgtattga     360

atcaagctat cacagaaaag gcattgggaa ttacaggtta tcgaccagat aagagtagtc     420

aaacaattaa cctgaatttt agagggaacg aaataacttt ctactataca ccgaaggtaa     480

aagatgttgt ttacaccatc aagtatgtct tgaaggataa cccagctatt gaggtagcac     540

ctacagttac tcgtaaggtt gatggaagta tcatccgtgc gaaagaagct gctgcagatg     600

tgaataaaca gcatttgcag agtcagtctg gtgttactgc tgaaatgctg gaaatggatt     660

actatccaac tgtaaatact caaagtttga ttctaacttc tagtgatgca aataatgtga     720

ttgtttttga atatgtcgga ttcgatactc aaacctttac ggtaaattat ttggatatgg     780

atggtaagaa aatcgatggt caagaaccat tggtagttta ccgaaagaaa ccaggtagtt     840

tcgtggtaga tcataagact ataaatggtt atacctatga tcattcgtta gacagtgaga     900

atgcagctga taagacagta taccgtgtaa gtaagggtgg tcacataacc attgatctct     960

attataagaa aaacctaaca ctgaccgcat tagataagtc caaagtttat gatggcactg    1020

ccctatcaag ttcgggtctg tctgatttaa atccttcgta cagttcagaa ctgaaaacag    1080

gtgacaaatt ggttgccatc agctatgatg gaagtcaaac aaatgctggt agtagcgcaa    1140

cgacacctaa acttgctaac atcagcaatg catctggtgc tgaccggacg aatttctatc    1200

acataaccgg cggccgc                                                   1217


<210> 7
<211> 544
<212> PRT
<213> Artificial Sequence

<220>
<221> source
<223> /note="Description of Artificial Sequence: Synthetic
      polypeptide"

<400> 7
Met Lys Leu Lys Val Thr Val Asn Gly Thr Ala Tyr Asp Val Asp Val
1               5                   10                  15


Asp Val Asp Lys Ser His Glu Asn Pro Met Gly Thr Ile Leu Phe Gly
            20                  25                  30

Gly Gly Thr Gly Gly Ala Pro Ala Pro Ala Ala Gly Gly Ala Gly Ala
        35                  40                  45

Gly Lys Ala Gly Glu Gly Glu Ile Pro Ala Pro Leu Ala Gly Thr Val
    50                  55                  60

Ser Lys Ile Leu Val Lys Glu Gly Asp Thr Val Lys Ala Gly Gln Thr
65                  70                  75                  80

Val Leu Val Leu Glu Ala Met Lys Met Glu Thr Glu Ile Asn Ala Pro
                85                  90                  95

Thr Asp Gly Lys Val Glu Lys Val Leu Val Lys Glu Arg Asp Ala Val
            100                 105                 110

Gln Gly Gly Gln Gly Leu Ile Lys Ile Gly Asp Leu Glu Leu Ile Glu
        115                 120                 125

Gly Arg Glu Ala Ser Ala Gly Ile Arg Asn Lys Met Pro Ala Asn Asn
    130                 135                 140

Leu Val Leu Tyr Ala Lys Trp Ile Ala Pro Lys Phe Lys Val Thr Phe
145                 150                 155                 160

Asp Ile Asn Gly Gly Asn Gly Thr Ala Pro Val Asp Gln Ser Val Glu
                165                 170                 175

Lys Tyr Lys Tyr Ala Lys Ala Pro Ala Asp Pro Thr Arg Glu His Tyr
            180                 185                 190

Asn Phe Leu Gly Trp Ser Thr Glu Asp Gly Lys Leu Tyr Ser Trp Ser
        195                 200                 205

Asp Gln Val Thr Lys Asp Val Lys Leu Thr Ala Lys Trp Glu Leu Lys
    210                 215                 220

Pro Leu Thr Tyr Thr Val Arg Tyr Leu Glu Ala Gly Thr Asn Thr Gln
225                 230                 235                 240

Leu Ala Ala Asp Lys Val Ile Thr Ser Pro Ala Leu Val Leu Asn Gln
                245                 250                 255

Ala Ile Thr Glu Lys Ala Leu Gly Ile Thr Gly Tyr Arg Pro Asp Lys
            260                 265                 270

Ser Ser Gln Thr Ile Asn Leu Asn Phe Arg Gly Asn Glu Ile Thr Phe
        275                 280                 285

Tyr Tyr Thr Pro Lys Val Lys Asp Val Val Tyr Thr Ile Lys Tyr Val
    290                 295                 300

Leu Lys Asp Asn Pro Ala Ile Glu Val Ala Pro Thr Val Thr Arg Lys
305                 310                 315                 320

Val Asp Gly Ser Ile Ile Arg Ala Lys Glu Ala Ala Ala Asp Val Asn
                325                 330                 335

Lys Gln His Leu Gln Ser Gln Ser Gly Val Thr Ala Glu Met Leu Glu
            340                 345                 350

Met Asp Tyr Tyr Pro Thr Val Asn Thr Gln Ser Leu Ile Leu Thr Ser
        355                 360                 365

Ser Asp Ala Asn Asn Val Ile Val Phe Glu Tyr Val Gly Phe Asp Thr
    370                 375                 380

Gln Thr Phe Thr Val Asn Tyr Leu Asp Met Asp Gly Lys Lys Ile Asp
385                 390                 395                 400

Gly Gln Glu Pro Leu Val Val Tyr Arg Lys Lys Pro Gly Ser Phe Val
                405                 410                 415

Val Asp His Lys Thr Ile Asn Gly Tyr Thr Tyr Asp His Ser Leu Asp
            420                 425                 430

Ser Glu Asn Ala Ala Asp Lys Thr Val Tyr Arg Val Ser Lys Gly Gly
        435                 440                 445

His Ile Thr Ile Asp Leu Tyr Tyr Lys Lys Asn Leu Thr Leu Thr Ala
    450                 455                 460

Leu Asp Lys Ser Lys Val Tyr Asp Gly Thr Ala Leu Ser Ser Ser Gly
465                 470                 475                 480

Leu Ser Asp Leu Asn Pro Ser Tyr Ser Ser Glu Leu Lys Thr Gly Asp
                485                 490                 495

Lys Leu Val Ala Ile Ser Tyr Asp Gly Ser Gln Thr Asn Ala Gly Ser
            500                 505                 510

Ser Ala Thr Thr Pro Lys Leu Ala Asn Ile Ser Asn Ala Ser Gly Ala
        515                 520                 525

Asp Arg Thr Asn Phe Tyr His Ile Thr Gly Gly Arg Asp Leu Val Leu
    530                 535                 540

<210> 8
<211> 2097
<212> DNA
<213> Streptococcus suis

<400> 8
gtgggaaaat gtataaatct atattttaag aaggagccaa ttatgagcaa gcagaaagtt      60

gtgtccagtt tattattaag cacagtcgta ttagggggat tagcctttta ttcaacgaca 120
acggttaaag cagaatcgct agaaccagat gttacatcag ttaatgcaag cgatgctact 180
aataaaccgg tagtaccaaa tgaagaggga ggcgactttt tagctgaaga ggagcttgaa 240
gatgatgaca ctttggaaga agaattaaat gagaaagccg aagaggttac tgagccatct 300
tcacctgaag cacttctcca acctcgtgcg atgatgagtg attctgaaac tagtggtatg 360
gaagaaattc cgatgaatga tgaaccttca gataatacgg aagaaaaggt agagaagcaa 420
cagtcgccat taattcaaac ctcaaatgca gattataaaa gtggtaaaga tcaagagaaa 480
cttaggacat cagtatctat aaatctgttg aaggcggaag aaggtcagat tcagtggaaa 540
gtaacttttg atacaagcga atggtctttt aatgttaaac atgggggtgt ttattttatt 600
cttccaaatg gtttggactt aacaaagatt gttgataata atcaacatga tataacagcc 660
tccttcccta cggatattaa tgattacaga aattctggtc aagaaaaata tcgtttcttt 720
agtagtaaac aaggtcttga caacgaaaat ggttttaatt ctcaatggaa ctggtctgct 780
ggtcaagcta atcctagtga aacagtaaac agttggaaat caggcaatcg tttgtctaag 840
atttatttca taaatcaaat aacagatact accgaattga cctatacttt aactgctaag 900
gtaactgaac caaatcagca atcattccct cttctggcag tgatgaagag ctttacgtat 960
acaaactcta agagtacaga ggttacttcg ctaggtgcac gtgagattac ccttgagaaa 1020
gaaaaaactc taccacctaa ggagaatcca aaaccagagc cagaagctcc aaaaccagac 1080
gctccacaag cgccatcagc accggaatct ccaacggaag aacctaagaa ggaagacgct 1140
ccagcgccat cgactccgga aaaacaacca gaagtaccgg aatcaccaaa cccagagaca 1200
ccagacgcgc catcgactcc aaaagatgag ccgcaagttc catcaatccc agaggagcaa 1260
ccaaaagaga ctccagcgcc agaagaacct aagaaggaag atactccgca aacaccacaa 1320
gcgccatcaa ctcctaaaga ggaagcaccg aaggaagaag ttccaacacc accggctcca 1380
tcagtaccag aagagcaacc aaaagaaact ccaacaccag aagttccaaa acaagaagat 1440
gttcaaccgg aagcgccaaa atctgataaa gtagaatctg acaaacaaat gccagaaact 1500
aagaaaccag atatgaagca accgaaggca gacgacatgc ctaaggaaca aaagccgaaa 1560
gccgacgagc caaaggcaga gcaaccacaa atggacaaac cacaaatgga agctcctaag 1620
aaagattcgg aagcgccaaa atctgataaa gtagaaactg acaagcaatt gccagaaact 1680
aagcaaccag atatgaagca accgaaggca gacgacatgc ctaaggaaca aaaaccgaaa 1740
gccgacgagc caaaggcaga gcaaccacaa atggacaaac cacaaatgga agctcctaag 1800
aaagattcgg aagcaccaaa atctgataaa gtagaaactg acaaaccaat gccagaaact 1860
aaacaaccag atatgaagca accgaaggca gataaaccag aagcagagaa agctcaaatg 1920
ccacggacag agggtatgaa gcctgaatct aaggcttcaa tgatgccaaa agcagaggca 1980
ccaaaagcaa ctttgccaaa tacaggcgaa gcaagtagcg caataggctg gctaggtggt 2040
gctttagcca ctcttgccac aggcttgtat ctattcaaaa acaaaaaaga agaatag 2097

<210> 9
<211> 698
<212> PRT
<213> Streptococcus suis

<400> 9
Met Gly Lys Cys Ile Asn Leu Tyr Phe Lys Lys Glu Pro Ile Met Ser
1               5                   10                  15

Lys Gln Lys Val Val Ser Ser Leu Leu Leu Ser Thr Val Val Leu Gly
            20                  25                  30

Gly Leu Ala Phe Tyr Ser Thr Thr Thr Val Lys Ala Glu Ser Leu Glu
        35                  40                  45

Pro Asp Val Thr Ser Val Asn Ala Ser Asp Ala Thr Asn Lys Pro Val
    50                  55                  60

Val Pro Asn Glu Glu Gly Gly Asp Phe Leu Ala Glu Glu Glu Leu Glu
65                  70                  75                  80

Asp Asp Asp Thr Leu Glu Glu Glu Leu Asn Glu Lys Ala Glu Glu Val
                85                  90                  95

Thr Glu Pro Ser Ser Pro Glu Ala Leu Leu Gln Pro Arg Ala Met Met
            100                 105                 110

Ser Asp Ser Glu Thr Ser Gly Met Glu Glu Ile Pro Met Asn Asp Glu
        115                 120                 125

Pro Ser Asp Asn Thr Glu Glu Lys Val Glu Lys Gln Gln Ser Pro Leu
    130                 135                 140

Ile Gln Thr Ser Asn Ala Asp Tyr Lys Ser Gly Lys Asp Gln Glu Lys
145                 150                 155                 160

Leu Arg Thr Ser Val Ser Ile Asn Leu Leu Lys Ala Glu Glu Gly Gln
                165                 170                 175

Ile Gln Trp Lys Val Thr Phe Asp Thr Ser Glu Trp Ser Phe Asn Val
            180                 185                 190

Lys His Gly Gly Val Tyr Phe Ile Leu Pro Asn Gly Leu Asp Leu Thr
        195                 200                 205

Lys Ile Val Asp Asn Asn Gln His Asp Ile Thr Ala Ser Phe Pro Thr
    210                 215                 220

Asp Ile Asn Asp Tyr Arg Asn Ser Gly Gln Glu Lys Tyr Arg Phe Phe
225                 230                 235                 240

Ser Ser Lys Gln Gly Leu Asp Asn Glu Asn Gly Phe Asn Ser Gln Trp
                245                 250                 255

Asn Trp Ser Ala Gly Gln Ala Asn Pro Ser Glu Thr Val Asn Ser Trp
            260                 265                 270

Lys Ser Gly Asn Arg Leu Ser Lys Ile Tyr Phe Ile Asn Gln Ile Thr
        275                 280                 285

Asp Thr Thr Glu Leu Thr Tyr Thr Leu Thr Ala Lys Val Thr Glu Pro
    290                 295                 300

Asn Gln Gln Ser Phe Pro Leu Leu Ala Val Met Lys Ser Phe Thr Tyr
305                 310                 315                 320

Thr Asn Ser Lys Ser Thr Glu Val Thr Ser Leu Gly Ala Arg Glu Ile
                325                 330                 335

Thr Leu Glu Lys Glu Lys Thr Leu Pro Pro Lys Glu Asn Pro Lys Pro
            340                 345                 350

Glu Pro Glu Ala Pro Lys Pro Asp Ala Pro Gln Ala Pro Ser Ala Pro
        355                 360                 365

Glu Ser Pro Thr Glu Glu Pro Lys Lys Glu Asp Ala Pro Ala Pro Ser
    370                 375                 380

Thr Pro Glu Lys Gln Pro Glu Val Pro Glu Ser Pro Asn Pro Glu Thr
385                 390                 395                 400

Pro Asp Ala Pro Ser Thr Pro Lys Asp Glu Pro Gln Val Pro Ser Ile
                405                 410                 415

Pro Glu Glu Gln Pro Lys Glu Thr Pro Ala Pro Glu Glu Pro Lys Lys
            420                 425                 430

Glu Asp Thr Pro Gln Thr Pro Gln Ala Pro Ser Thr Pro Lys Glu Glu
        435                 440                 445

Ala Pro Lys Glu Glu Val Pro Thr Pro Pro Ala Pro Ser Val Pro Glu
    450                 455                 460

Glu Gln Pro Lys Glu Thr Pro Thr Pro Glu Val Pro Lys Gln Glu Asp
465                 470                 475                 480

Val Gln Pro Glu Ala Pro Lys Ser Asp Lys Val Glu Ser Asp Lys Gln
                485                 490                 495

Met Pro Glu Thr Lys Lys Pro Asp Met Lys Gln Pro Lys Ala Asp Asp
            500                 505                 510

Met Pro Lys Glu Gln Lys Pro Lys Ala Asp Glu Pro Lys Ala Glu Gln
        515                 520                 525

Pro Gln Met Asp Lys Pro Gln Met Glu Ala Pro Lys Lys Asp Ser Glu
    530                 535                 540

Ala Pro Lys Ser Asp Lys Val Glu Thr Asp Lys Gln Leu Pro Glu Thr
545                 550                 555                 560

Lys Gln Pro Asp Met Lys Gln Pro Lys Ala Asp Asp Met Pro Lys Glu
                565                 570                 575

Gln Lys Pro Lys Ala Asp Glu Pro Lys Ala Glu Gln Pro Gln Met Asp
            580                 585                 590

Lys Pro Gln Met Glu Ala Pro Lys Lys Asp Ser Glu Ala Pro Lys Ser
        595                 600                 605

Asp Lys Val Glu Thr Asp Lys Pro Met Pro Glu Thr Lys Gln Pro Asp
    610                 615                 620

Met Lys Gln Pro Lys Ala Asp Lys Pro Glu Ala Glu Lys Ala Gln Met
625                 630                 635                 640

Pro Arg Thr Glu Gly Met Lys Pro Glu Ser Lys Ala Ser Met Met Pro
                645                 650                 655

Lys Ala Glu Ala Pro Lys Ala Thr Leu Pro Asn Thr Gly Glu Ala Ser
            660                 665                 670

Ser Ala Ile Gly Trp Leu Gly Gly Ala Leu Ala Thr Leu Ala Thr Gly
        675                 680                 685

Leu Tyr Leu Phe Lys Asn Lys Lys Glu Glu
    690                 695

<210> 10
<211> 1309
<212> DNA
<213> Artificial Sequence

<220>
<221> source
<223> /note="Description of Artificial Sequence: Synthetic
      polynucleotide"

<400> 10
gggatccggg gtcaagctaa tcctagtgaa acagtaaaca gttggaaatc aggcaatcgt      60

ttgtctaaga tttatttcat aaatcaaata acagatacta ccgaattgac ctatacttta     120

actgctaagg taactgaacc aaatcagcaa tcattccctc ttctggcagt gatgaagagc     180

tttacgtata caaactctaa gagtacagag gttacttcgc taggtgcacg tgagattacc     240

cttgagaaag aaaaaactct accacctaag gagaatccaa aaccagagcc agaagctcca     300

aaaccagacg ctccacaagc gccatcagca ccggaatctc caacggaaga acctaagaag     360

gaagacgctc cagcgccatc gactccggaa aaacaaccag aagtaccgga atcaccaaac     420

ccagagacac cagacgcgcc atcgactcca aaagatgagc cgcaagttcc atcaatccca 480

gaggagcaac caaaagagac tccagcgcca gaagaaccta agaaggaaga tactccgcaa 540

acaccacaag cgccatcaac tcctaaagag gaagcaccga aggaagaagt tccaacacca 600

ccggctccat cagtaccaga agagcaacca aaagaaactc caacaccaga agttccaaaa 660

caagaagatg ttcaaccgga agcgccaaaa tctgataaag tagaatctga caaacaaatg 720

ccagaaacta agaaaccaga tatgaagcaa ccgaaggcag acgacatgcc taaggaacaa 780

aagccgaaag ccgacgagcc aaaggcagag caaccacaaa tggacaaacc acaaatggaa 840

gctcctaaga aagattcgga agcgccaaaa tctgataaag tagaaactga caagcaattg 900

ccagaaacta agcaaccaga tatgaagcaa ccgaaggcag acgacatgcc taaggaacaa 960

aaaccgaaag ccgacgagcc aaaggcagag caaccacaaa tggacaaacc acaaatggaa 1020

gctcctaaga aagattcgga agcaccaaaa tctgataaag tagaaactga caaaccaatg 1080

ccagaaacta aacaaccaga tatgaagcaa ccgaaggcag ataaaccaga agcagagaaa 1140

gctcaaatgc cacggacaga gggtatgaag cctgaatcta aggcttcaat gatgccaaaa 1200

gcagaggcac caaaagcaac tttgccaaat acaggcgaag caagtagcgc aataggctgg 1260

ctaggtggtg ctttagccac tcttgccaca ggcttgtatc taccccggg 1309

<210> 11
<211> 577
<212> PRT
<213> Artificial Sequence

<220>
<221> source
<223> /note="Description of Artificial Sequence: Synthetic
      polypeptide"

<400> 11
Met Lys Leu Lys Val Thr Val Asn Gly Thr Ala Tyr Asp Val Asp Val
1               5                   10                  15

Asp Val Asp Lys Ser His Glu Asn Pro Met Gly Thr Ile Leu Phe Gly
            20                  25                  30

Gly Gly Thr Gly Gly Ala Pro Ala Pro Ala Ala Gly Gly Ala Gly Ala
        35                  40                  45

Gly Lys Ala Gly Glu Gly Glu Ile Pro Ala Pro Leu Ala Gly Thr Val
    50                  55                  60

Ser Lys Ile Leu Val Lys Glu Gly Asp Thr Val Lys Ala Gly Gln Thr
65                  70                  75                  80

Val Leu Val Leu Glu Ala Met Lys Met Glu Thr Glu Ile Asn Ala Pro
                85                  90                  95

Thr Asp Gly Lys Val Glu Lys Val Leu Val Lys Glu Arg Asp Ala Val
            100                 105                 110

Gln Gly Gly Gln Gly Leu Ile Lys Ile Gly Asp Leu Glu Leu Ile Glu
        115                 120                 125

Gly Arg Glu Ala Ser Ala Gly Ile Arg Gly Gln Ala Asn Pro Ser Glu
    130                 135                 140

Thr Val Asn Ser Trp Lys Ser Gly Asn Arg Leu Ser Lys Ile Tyr Phe
145                 150                 155                 160

Ile Asn Gln Ile Thr Asp Thr Thr Glu Leu Thr Tyr Thr Leu Thr Ala
                165                 170                 175

Lys Val Thr Glu Pro Asn Gln Gln Ser Phe Pro Leu Leu Ala Val Met
            180                 185                 190

Lys Ser Phe Thr Tyr Thr Asn Ser Lys Ser Thr Glu Val Thr Ser Leu
        195                 200                 205

Gly Ala Arg Glu Ile Thr Leu Glu Lys Glu Lys Thr Leu Pro Pro Lys
    210                 215                 220

Glu Asn Pro Lys Pro Glu Pro Glu Ala Pro Lys Pro Asp Ala Pro Gln
225                 230                 235                 240

Ala Pro Ser Ala Pro Glu Ser Pro Thr Glu Glu Pro Lys Lys Glu Asp
                245                 250                 255

Ala Pro Ala Pro Ser Thr Pro Glu Lys Gln Pro Glu Val Pro Glu Ser
            260                 265                 270

Pro Asn Pro Glu Thr Pro Asp Ala Pro Ser Thr Pro Lys Asp Glu Pro
        275                 280                 285

Gln Val Pro Ser Ile Pro Glu Glu Gln Pro Lys Glu Thr Pro Ala Pro
    290                 295                 300

Glu Glu Pro Lys Lys Glu Asp Thr Pro Gln Thr Pro Gln Ala Pro Ser
305                 310                 315                 320

Thr Pro Lys Glu Glu Ala Pro Lys Glu Glu Val Pro Thr Pro Pro Ala
                325                 330                 335

Pro Ser Val Pro Glu Glu Gln Pro Lys Glu Thr Pro Thr Pro Glu Val
            340                 345                 350

Pro Lys Gln Glu Asp Val Gln Pro Glu Ala Pro Lys Ser Asp Lys Val
        355                 360                 365

Glu Ser Asp Lys Gln Met Pro Glu Thr Lys Lys Pro Asp Met Lys Gln
    370                 375                 380

Pro Lys Ala Asp Asp Met Pro Lys Glu Gln Lys Pro Lys Ala Asp Glu
385                 390                 395                 400

Pro Lys Ala Glu Gln Pro Gln Met Asp Lys Pro Gln Met Glu Ala Pro
                405                 410                 415

Lys Lys Asp Ser Glu Ala Pro Lys Ser Asp Lys Val Glu Thr Asp Lys
            420                 425                 430

Gln Leu Pro Glu Thr Lys Gln Pro Asp Met Lys Gln Pro Lys Ala Asp
        435                 440                 445

Asp Met Pro Lys Glu Gln Lys Pro Lys Ala Asp Glu Pro Lys Ala Glu
    450                 455                 460

Gln Pro Gln Met Asp Lys Pro Gln Met Glu Ala Pro Lys Lys Asp Ser
465                 470                 475                 480

Glu Ala Pro Lys Ser Asp Lys Val Glu Thr Asp Lys Pro Met Pro Glu
                485                 490                 495

Thr Lys Gln Pro Asp Met Lys Gln Pro Lys Ala Asp Lys Pro Glu Ala
            500                 505                 510

Glu Lys Ala Gln Met Pro Arg Thr Glu Gly Met Lys Pro Glu Ser Lys
        515                 520                 525

Ala Ser Met Met Pro Lys Ala Glu Ala Pro Lys Ala Thr Leu Pro Asn
    530                 535                 540

Thr Gly Glu Ala Ser Ser Ala Ile Gly Trp Leu Gly Gly Ala Leu Ala
545                 550                 555                 560

Thr Leu Ala Thr Gly Leu Tyr Leu Pro Arg Gly Gly Arg Asp Leu Val
                565                 570                 575

Leu

<210> 12
<211> 23
<212> DNA
<213> Artificial Sequence

<220>
<221> source
<223> /note="Description of Artificial Sequence: Synthetic
      primer"

<400> 12
cgcggatccg ggagtctgct agc                                              23

<210> 13
<211> 31
<212> DNA

<213> Artificial Sequence

<220>
<221> source
<223> /note="Description of Artificial Sequence: Synthetic
      primer"

<400> 13
gagtcattct gcggccgcca aaggttttcc g                                    31


<210> 14
<211> 24
<212> DNA
<213> Artificial Sequence

<220>
<221> source
<223> /note="Description of Artificial Sequence: Synthetic
      primer"

<400> 14
cgcggatccg gaataagatg cctg                                            24


<210> 15
<211> 32
<212> DNA
<213> Artificial Sequence

<220>
<221> source
<223> /note="Description of Artificial Sequence: Synthetic
      primer"

<400> 15
gagtcattct gcggccgccg gttatgtgat ag                                   32


<210> 16
<211> 24
<212> DNA
<213> Artificial Sequence

<220>
<221> source
<223> /note="Description of Artificial Sequence: Synthetic
      primer"

<400> 16
cgcggatccg gggtcaagct aatc                                            24


<210> 17
<211> 25
<212> DNA
<213> Artificial Sequence

<220>
<221> source
<223> /note="Description of Artificial Sequence: Synthetic
      primer"

<400> 17
tccggacccg gggtagatac aagcc                                           25

## Description

### Technical Field

The present invention refers to the field of infectious diseases and more in particular it refers to protein Ssu05_0473 and protein Ssu05_0272 or immunogenic derivatives (also referred to as polypeptide or polypeptides of the invention) thereof for use as a medicament, in particular for use as a medicament against Streptococcus suis colonization or infection in humans and animals.

The present invention also refers to a vaccine comprising at least one of said proteins or immunogenic derivatives thereof, to a method for the diagnosis of bacterial infections using said proteins or immunogenic derivatives thereof and to a kit for the diagnosis of Streptococcus suis infections.

### Background of the invention

Streptococcus suis is an important pathogen associated with a wide range of diseases in pigs, including meningitis, septicaemia, pneumonia, endocarditis, and arthritis. S. suis can be transmitted to human beings by direct contact (Lun et al, 2007). Based on the capsular polysaccharides, 35 serotypes have been identified (types 1-34 and 1/2).

The resurgence of infectious diseases caused by Streptococcus suis has heightened public concern, largely because resistance to certain antibiotics has recently emerged. In July 2005, a large outbreak of human S. suis infection occurred in Sichuan province, China, accompanied by several sporadic cases in other provinces. This was the third reported outbreak of human S. suis infections that has occurred in China, with two earlier outbreaks occurring in Jiangsu province in 1998 and 1999. In the past 8 years in China, at least 237 people have been infected with S. suis and 53 of them died. The repeated intensive outbreaks of human S. suis infection have raised great public concern worldwide regarding it as an emerging zoonotic agent. Therefore, research on other treatments, mainly vaccines, are key to overcome drug resistance.

To date, several approaches have been applied to develop vaccines against this pathogen. Nevertheless, the success achieved has been very limited due to serotype-or strain-dependent protection. Using the capsular polysaccharide as an antigen, the results have been unsatisfactory due to poor immunogenicity. Then, interest has been focused on surface proteins as potential vaccine candidates. Some of them have been proposed and tested, as suilysin, muramidase-released protein (MRP) or extracellular protein factor, but their use is limited because an important number of virulent strains in some geographical regions do not express these proteins (Li et al, 2006 and 2007). More recently, protein Sao has been proposed as a new protein candidate (Li et al, 2006 and 2007).

There is a need of new vaccine compositions based on new antigens, or fragments thereof, capable of recognising a higher number of serotypes and induce immune response in animals or humans infected by virulent strains of such serotypes.

During the last years, one of the inventors and colleagues have set up and applied a novel proteomics-based technology for the massive identification of surface protein antigens in several Gram-positive pathogenic bacteria (Rodríguez-Ortega et al 2006, Nat. Biotechnol. 24 (2): 191-197; Rodríguez-Ortega et al 2008, BMC Genomics 9: art 588). In 2007, the present inventors focused their attention on the identification of surface proteins from Streptococcus suis using the previously cited proteomic strategy. For this microorganism, two genomes have been very recently sequenced and annotated, those corresponding to strains 05ZYH33 and 98HAH33 (Chen et al, PLoS ONE 2007, 2(3): e315).

The method for the identification of antigens or their fragments of Streptococcus suis surface proteins, by means of identification of peptides generated after protease digestion of their surface-exposed domains, comprises the following steps:
a) Bacterial culture in Todd-Hewitt Broth until mid-exponential phase is reached;
b) Centrifugation, discarding of the culture medium (supernatant) and washing of bacteria (pellet) three times with PBS;
c) Bacterial resuspension in isotonic buffer, which contains the protease;
d) Digestion at 37 °C for 30 minutes, with very gentle shaking;
e) Centrifugation to recover the supernatant, which contains the released peptides;
f) Sample cleaning/desalting by employing reversed-phase chromatography cartridges, and
g) Separation and identification of peptides by 2-D LC/MS/MS.

Briefly, such a strategy consists of incubating live cells with proteases (trypsin, proteinase K) in order to cleave the exposed domains of surface proteins. The peptides generated from shaved bacterial cells are recovered and then analysed by twodimensional liquid chromatography coupled to tandem mass spectrometry (2-D LC/MS/MS). The mass spectra thus obtained are used for database searching and peptide sequence identification. This approach allows the recovery of a fraction of surface proteins in a fast and reliable way without cytoplasmic contamination, as cells remain intact. Then, false positives are discarded. Moreover, the identified proteins are contrasted by computational analysis and their sequences are inspected to correct possible errors in function prediction.

Surface-exposed Streptococcus suis antigens as defined below can be identified using a simple and cost-effective approach which combines liquid chromatography and tandem mass spectrometry. The surface-exposed S. suis antigens can be used as active agents in compositions for preventing and for treating S. suis infections.

### Disclosure of Invention

By combining bioinformatic analysis, mainly algorithms for subcellular location prediction, and experimental work, i.e. the above cited proteomic approach, the inventors of the present invention have found 3 proteins for which the predicted (cytoplasmic) functions did not correspond neither to protein sequence features, determined by bioinformatic analysis, as the presence of cell-wall anchoring LPXTG (SEQ ID NO: 1) motifs, nor to surface localisation according to proteomics identification, which can be also corroborated by FACS analysis.

It has been then found that the predicted and primarily annotated functions of these 3 proteins were wrong.

In particular it has been found that two of these 3 proteins, Protein Ssu05_0473 and protein Ssu05_0272, and their fragments are immunogenic and show a clear protective activity against S. suis infection.

Therefore, object of the present invention are proteins Ssu05_0473 and Ssu05_0272 or immunogenic derivatives thereof, in particular the fragment aa853-aa1252 of protein Ssu05_0473, for use as a medicament.

The polynucleotide sequence (SEQ ID NO: 2) of the whole gene coding for Ssu05_0473 is:

gtgcaagaaacaccagtaacaacggaaattagtaaattaacagaaccgatggtgacaact
cagcaaactccgatagtgggtagctctgctgatgaagttcaatctcctgcgacagaaacg
gttcctgaccctgctgagcaagtgtcaacagagtctgctagcaaccccagaccgaccaa
gaacggattactgagccaactaccatagttcacaaaacgaatgattatgaggtgactgca
acatttgatgcgagtgctcaatttcccaaagatgttgagtttaatgttagagaactagat
agccaatcagaagaatatcggacacattatgaaaaaccaaggagaagcttggagtaaaa
aaacttgtatatgcccgattctttgatattcatttttcatcacagggccaagaggtcgag
cctgcctcccctgtgaagattacgattagaaacaaggaattagttcaacggccagatgat
agtaaactgaaaattgtccactttgaagagaatagccagatagaagttttagaggccgaa
acgaaggaaaatgaacaacaaatctccgagatcagttttgacgctagccatttttctgtt
tatggagatgttttagcaaactattatactgtcaaatttacctacactgacacagcaggt
caagaacaagtcattacgagtttgattgataagactgtgggacttcacttggtagtttg
cctgaagctccttttaaaaacggttatgtatttctcattggattaataaagatactggt
gatcggattgatgaaaatacggtgataactggcgacctgactgctgtagcagtatttaat
aatatcaatatctacaccattgatgtcacgtatttctaccacaatgtttctaaaaaccaa
gatattacgattgataaggaaacggtgcaactggaagcaagtgatgctccatatcaactg
acaccacctgcttctactgaggtgagcaacaaagaggatacaactttaccagcagatgct
atttactaccctgaaaaaccacttcttgagtttacagcagatcaactagagcgactggac
gtagcagatggagtggatgataattatattagcgaaagagtacaatatgttccttacaat
gcagagtatgatgtcatttataggctaaagaatttgacggggtcaggttattctgatatc
cagactgttcatgtccgtggggtactgggttcaactgttacaccgcaaattctaacctac
aactatgctgactttgaacgtgctgatacagcaaagattgagaatactagcggccaaaaa
ttgtatgtttattatacacggaaaacctttactctttcttataattccaatggcggtagt
aatattccccaacaacaggtctatacgatagccagattaagatttctacaactactcca
acaaaagagggggtatacatttgaaggttggtatgataatcctggactgactggaaacaag
attacaggctctctcaccttaaagaaagatacaactttgtatgcgaaatggacaccgaac
agggttcgctacatcattgggtattacaaggaagtctatgacaacaacactggaacaacc
tcctatgtatatgatagttcggcagctggttggggtctggttgggactactgtaaaagga
acagattcaaatattggacctcctccgccaacccaaacaaccattggcagcattggctac
gagaaggatgttactaaaagtgctgcttcaagtattgaaattgcagctgatggaacatct
gttttgaaagtttactataaactgaagcgctacacttttgaattttcttaaatgataac
aatgccaatatcaccataaatggtaagacctactctgataggaataggtacatgatacgt
gatgttgtgcttggtcaagatgtctcaaagttatggccagcttctataacaaatcctaaa
gaagtatacgatgataaagtctactatgatagctatggtaggccgataagatactttttt
gactactggatttttgggaatctactcttcaaaacagatcggttcgaagttgttgagtct
atgttgccaacgagtggaacaaccagtaagttcacagcatattggaatacccaaggtaca
caggctaacgttgaatattggttacaacaaccggatggttcttataagaagtctgatata
tatagtcaatctttcctaaagactgcaggactgctaccgaaagaaatttttggatattct
tattatacagatgctagttttactgaagcaggcattaggtattacggaactcaaggaaat
acatatcgtttctactataaacgccaagccttcaaaattgactattattacaatagtact
gggttagattcaaagagtaacattctctttgagagtaacatcaatacagcaacttataat
taccaaccaccacgttctactggagttgatgctgattatacatggggtggatggtataca
gattcgttattgacagagaagtataactttaataagatgcctgccaataacttggttctc

tatgctaagtggattgctccaaaatttaaggttacttttgatatcaacggtggtaatgga
actgcccctgtcgatcaaagtgttgaaaaatacaaatacgctaaggcccctgctgatcca
acgagggaacattacaatttccttggttggtctacagaagatgggaaactctatagttgg
agcgaccaagtaaccaaggatgtcaaattgactgcgaagtgggaattgaaaccattaact
tatactgttcgctatcttgaagcaggtacaaatacgcaattggctgctgataaagtgatt
acgagtccagcgcttgtattgaatcaagctatcacagaaaaggcattgggaattacaggt
tatcgaccagataagagtagtcaaacaattaacctgaattttagagggaacgaaataact
ttctactatacaccgaaggtaaaagatgttgtttacaccatcaagtatgtcttgaaggat
aacccagctattgaggtagcacctacagttactcgtaaggttgatggaagtatcatccgt
gcgaaagaagctgctgcagatgtgaataaacagcatttgcagagtcagtctggtgttact
gctgaaatgctggaaatggattactatccaactgtaaatactcaaagtttgattctaact
tctagtgatgcaaataatgtgattgtttttgaatatgtcggattcgatactcaaaccttt
acggtaaattatttggatatggatggtaagaaaatcgatggtcaagaaccattggtagtt
taccgaaagaaaccaggtagtttcgtggtagatcataagactataaatggttatacctat
gatcattcgttagacagtgagaatgcagctgataagacagtataccgtgtaagtaagggt
ggtcacataaccattgatctctattataagaaaaacctaacactgaccgcattagataag
tccaaagtttatgatggcactgccctatcaagttcgggtctgtctgatttaaatccttcg
tacagttcagaactgaaaacaggtgacaaattggttgccatcagctatgatggaagtcaa
acaaatgctggtagtagcgcaacgacacctaaacttgctaacatcagcaatgcatctggt
gctgaccggacgaatttctatcacataacctatcaaccgggtaacttaagagttgaccaa
cagccagttgtggttgtcatcgacggtgaaaagaaatcgaaaatctatgatggcaaagca
gaagcagttggttataaggtgacggaaataagagattcaagcggtctctataaaaaaaca
gatatcaaatttaatggttcaagttcagagcagtcggttaagaaaaaagatgcaggaatt
tatccactccttttaagtaatagattctcaaataccaatcagaatttccaagtagatttc
ttgatttctgatggggaattaagaatagatagacggcaactgaccataacatctgactcc
gctagcaaggcttatgacggtagcgagcttcgtgctgacaatatcacagtctctgtacct
gctggtgttgactacacaggttttgtcgatggcgaaggattataccaaccttcttaggc
ggtccaatcgattctggaatgatgccaaacttctttgaatatgctctaaaatctggcacg
aatttagctaattatgagttagatatccagtttggccagctcaaggtcaaagaagtcatc
aagattcagaaaacagacctggcctggcaggccctttcaggtggtaagtttgaattgacc
aagtgggatggtttgaactgggctcaggtagatggggtgcaggaatttgccattaccagc
aaagatggtattcggataccggtaggcttagaagcaggtcgctaccgtctgcaagaattg
gcagcacctgatggttttatcgtcttggatagctatatctacttcagcatcaaggaaatc
tttaacgaagataagacatcttccttctacacagtttccttgtctgatgaagcaggcaag
gatgctagtccggaacgagcaacgcttaccaaggttagtggtgatgccagtcatcgcatc
caagtggccaatgagcagggcagagccttgccaagcacaggcggagatggacagaagtgg
tttattctttctggccttgtcctcatagccatctcctatctgatgcatctgtttgttcaa
cgtcgaaggtaa

The amino acid sequence (SEQ ID NO: 3) coded by the above gene is:

MQETPVTTEISKLTEPMVTTQQTPIVGSSADEVQSPATETVPDPAEQVSTESASKPQTDQERITEPTTIV

HKTNDYEVTATFDASAQFPKDVEFNVRELDSQSEEYRTHYEKTKEKLGVKKLVYARFFDIHFSSQGQEVE

PASPVKITIRNKELVQRPDDSKLKIVHFEENSQIEVLEAETKENEQQISEISFDASHFSVYGDVLANYYT

VKFTYTDTAGQEQVITSLIDKTVGTSLGSLPEAPFKNGYVFSHWINKDTGDRIDENTVITGDLTAVAVFN

NINIYTIDVTYFYHNVSKNQDITIDKETVQLEASDAPYQLTPPASTEVSNKEDTTLPADAIYYPEKPLLE

FTADQLERLDVADGVDDNYISERVQYVPYNAEYDVIYRLKNLTGSGYSDIQTVHVRGVLGSTVTPQILTY

NYADFERADTAKIENTSGQKLYVYYTRKTFTLSYNSNGGSNIPQQQGLYDSQIKISTTTPTKEGYTFEGW
YDNPGLTGNKITGSLTLKKDTTLYAKWTPNRVRYIIGYYKEVYDNNTGTTSYVYDSSAAGWGLVGTTVKG
TDSNIGPPPPTQTTIGSIGYEKDVTKSAASSIEIAADGTSVLKVYYKLKRYTFEFFLNDNNANITINGKT
YSDRNRYMIRDVVLGQDVSKLWPASITNPKEVYDDKVYYDSYGRPIRYFFDYWIFGNLLFKTDRFEVVES
MLPTSGTTSKFTAYWNTQGTQANVEYWLQQPDGSYKKSDIYSQSFLKTAGLLPKEIFGYSYYTDASFTEA
GIRYYGTQGNTYRFYYKRQAFKIDYYYNSTGLDSKSNILFESNINTATYNYQPPRSTGVDADYTWGGWYT
DSLLTEKYNFNKMPANNLVLYAKWIAPKFKVTFDINGGNGTAPVDQSVEKYKYAKAPADPTREHYNFLGW
STEDGKLYSWSDQVTKDVKLTAKWELKPLTYTVRYLEAGTNTQLAADKVITSPALVLNQAITEKALGITG
YRPDKSSQTINLNFRGNEITFYYTPKVKDVVYTIKYVLKDNPAIEVAPTVTRKVDGSIIRAKEAAADVNK
QHLQSQSGVTAEMLEMDYYPTVNTQSLILTSSDANNVIVFEYVGFDTQTFTVNYLDMDGKKIDGQEPLVV
YRKKPGSFVVDHKTINGYTYDHSLDSENAADKTVYRVSKGGHITIDLYYKKNLTLTALDKSKVYDGTALS
SSGLSDLNPSYSSELKTGDKLVAISYDGSQTNAGSSATTPKLANISNASGADRTNFYHITYQPGNLRVDQ
QPVVVVIDGEKKSKIYDGKAEAVGYKVTEIRDSSGLYKKTDIKFNGSSSEQSVKKKDAGIYPLLLSNRFS
NTNQNFQVDFLISDGELRIDRRQLTITSDSASKAYDGSELRADNITVSVPAGVDYTGFVDGEGIIPTFLG
GPIDSGMMPNFFEYALKSGTNLANYELDIQFGQLKVKEVIKIQKTDLAWQALSGGKFELTKWDGLNWAQV
DGVQEFAITSKDGIRIPVGLEAGRYRLQELAAPDGFIVLDSYIYFSIKEIFNEDKTSSFYTVSLSDEAGK
DASPERATLTKVSGDASHRIQVANEQGRALPSTGGDGQKWFILSGLVLIAISYLMHLFVQRRR

Two fragments of the above coding gene were cloned, called FA1 and FA3. The nucleotide sequence (SEQ ID NO: 4) corresponding to fragment FA1 is:

GGATCCGGGAGTCTGCTAGCAAACCCCAGACCGACCAAGAACGGATTACTGAGCCAACTACCATAG
TTCACAAAACGAATGATTATGAGGTGACTGCAACATTTGATGCGAGTGCTCAATTTCCCAAAGATG
TTGAGTTTAATGTTAGAGAACTAGATAGCCAATCAGAAGAATATCGGACACATTATGAAAAAACCA
AGGAGAAGCTTGGAGTAAAAAAACTTGTATATGCCCGATTCTTTGATATTCATTTTTCATCACAGG
GCCAAGAGGTCGAGCCTGCCTCCCCTGTGAAGATTACGATTAGAAACAAGGAATTAGTTCAACGGC
CAGATGATAGTAAACTGAAAATTGTCCACTTTGAAGAGAATAGCCAGATAGAAGTTTTAGAGGCCG
AAACGAAGGAAAATGAACAACAAATCTCCGAGATCAGTTTTGACGCTAGCCATTTTTCTGTTTATG
GAGATGTTTTAGCAAACTATTATACTGTCAAATTTACCTACACTGACACAGCAGGTCAAGAACAAG
TCATTACGAGTTTGATTGATAAGACTGTGGGGACTTCACTTGGTAGTTTGCCTGAAGCTCCTTTTA
AAAACGGTTATGTATTTTCTCATTGGATTAATAAAGATACTGGTGATCGGATTGATGAAAATACGG
TGATAACTGGCGACCTGACTGCTGTAGCAGTATTTAATAATATCAATATCTACACCATTGATGTCA
CGTATTTCTACCACAATGTTTCTAAAAACCAAGATATTACGATTGATAAGGAAACGGTGCAACTGG
AAGCAAGTGATGCTCCATATCAACTGACACCACCTGCTTCTACTGAGGTGAGCAACAAAGAGGATA
CAACTTTACCAGCAGATGCTATTTACTACCCTGAAAAACCACTTCTTGAGTTTACAGCAGATCAAC
TAGAGCGACTGGACGTAGCAGATGGAGTGGATGATAATTATATTAGCGAAAGAGTACAATATGTTC
CTTACAATGCAGAGTATGATGTCATTTATAGGCTAAAGAATTTGACGGGGTCAGGTTATTCTGATA
TCCAGACTGTTCATGTCCGTGGGGTACTGGGTTCAACTGTTACACCGCAAATTCTAACCTACAACT

ATGCTGACTTTGAACGTGCTGATACAGCAAAGATTGAGAATACTAGCGGCCAAAAATTGTATGTTT
ATTATACACGGAAAACCTTTgGCGGCCGC

The amino acid sequence (SEQ ID NO: 5) coded by the above nucleotide sequence is:

MKLKVTVNGTAYDVDVDVDKSHENPMGTILFGGGTGGAPAPAAGGAGAGKAGEGEIPAPLAGTVSK
ILVKEGDTVKAGQTVLVLEAMKMETEINAPTDGKVEKVLVKERDAVQGGQGLIKIGDLELIEGREA
SAGIRESASKPQTDQERITEPTTIVHKTNDYEVTATFDASAQFPKDVEFNVRELDSQSEEYRTHYE
KTKEKLGVKKLVYARFFDIHFSSQGQEVEPASPVKITIRNKELVQRPDDSKLKIVHFEENSQIEVL
EAETKENEQQISEISFDASHFSVYGDVLANYYTVKFTYTDTAGQEQVITSLIDKTVGTSLGSLPEA
PFKNGYVFSHWINKDTGDRIDENTVITGDLTAVAVFNNINIYTIDVTYFYHNVSKNQDITIDKETV
QLEASDAPYQLTPPASTEVSNKEDTTLPADAIYYPEKPLLEFTADQLERLDVADGVDDNYISERVQ
YVPYNAEYDVIYRLKNLTGSGYSDIQTVHVRGVLGSTVTPQILTYNYADFERADTAKIENTSGQKL
YVYYTRKTFGGRDLVL

Theoretical pl/Mw: 4.70 / 60039.82. The molecular weight (MW) of the recombinant fragment determined by SDS-PAGE corresponded to that calculated from the sum of the amino acid residues (this is valid for the rest of recombinant fragments described in this application).

The nucleotide sequence (SEQ ID NO: 6) corresponding to fragment FA3 is:

GGATCCGGAATAAGATGCCTGCCAATAACTTGGTTCTCTATGCTAAGTGGATTGCTCCAAAATTTA
AGGTTACTTTTGATATCAACGGTGGTAATGGAACTGCCCCTGTCGATCAAAGTGTTGAAAAATACA
AATACGCTAAGGCCCCTGCTGATCCAACGAGGGAACATTACAATTTCCTTGGTTGGTCTACAGAAG
ATGGGAAACTCTATAGTTGGAGCGACCAAGTAACCAAGGATGTCAAATTGACTGCGAAGTGGGAAT
TGAAACCATTAACTTATACTGTTCGCTATCTTGAAGCAGGTACAAATACGCAATTGGCTGCTGATA
AAGTGATTACGAGTCCAGCGCTTGTATTGAATCAAGCTATCACAGAAAAGGCATTGGGAATTACAG
GTTATCGACCAGATAAGAGTAGTCAAACAATTAACCTGAATTTTAGAGGGAACGAAATAACTTTCT
ACTATACACCGAAGGTAAAAGATGTTGTTTACACCATCAAGTATGTCTTGAAGGATAACCCAGCTA
TTGAGGTAGCACCTACAGTTACTCGTAAGGTTGATGGAAGTATCATCCGTGCGAAAGAAGCTGCTG
CAGATGTGAATAAACAGCATTTGCAGAGTCAGTCTGGTGTTACTGCTGAAATGCTGGAAATGGATT
ACTATCCAACTGTAAATACTCAAAGTTTGATTCTAACTTCTAGTGATGCAAATAATGTGATTGTTT
TTGAATATGTCGGATTCGATACTCAAACCTTTACGGTAAATTATTTGGATATGGATGGTAAGAAAA
TCGATGGTCAAGAACCATTGGTAGTTTACCGAAAGAAACCAGGTAGTTTCGTGGTAGATCATAAGA
CTATAAATGGTTATACCTATGATCATTCGTTAGACAGTGAGAATGCAGCTGATAAGACAGTATACC
GTGTAAGTAAGGGTGGTCACATAACCATTGATCTCTATTATAAGAAAAACCTAACACTGACCGCAT
TAGATAAGTCCAAAGTTTATGATGGCACTGCCCTATCAAGTTCGGGTCTGTCTGATTTAAATCCTT
CGTACAGTTCAGAACTGAAAACAGGTGACAAATTGGTTGCCATCAGCTATGATGGAAGTCAAACAA
ATGCTGGTAGTAGCGCAACGACACCTAAACTTGCTAACATCAGCAATGCATCTGGTGCTGACCGGA
CGAATTTCTATCACATAACCgGCGGCCGC

The amino acid sequence (SEQ ID NO: 7) coded by the above nucleotide sequence is:

MKLKVTVNGTAYDVDVDVDKSHENPMGTILFGGGTGGAPAPAAGGAGAGKAGEGEIPAPLAGTV
SKILVKEGDTVKAGQTVLVLEAMKMETEINAPTDGKVEKVLVKERDAVQGGQGLIKIGDLELIE
GREASAGIRNKMPANNLVLYAKWIAPKFKVTFDINGGNGTAPVDQSVEKYKYAKAPADPTREHY
NFLGWSTEDGKLYSWSDQVTKDVKLTAKWELKPLTYTVRYLEAGTNTQLAADKVITSPALVLNQ
AITEKALGITGYRPDKSSQTINLNFRGNEITFYYTPKVKDVVYTIKYVLKDNPAIEVAPTVTRK

VDGSIIRAKEAAADVNKQHLQSQSGVTAEMLEMDYYPTVNTQSLILTSSDANNVIVFEYVGFDT
QTFTVNYLDMDGKKIDGQEPLVVYRKKPGSFVVDHKTINGYTYDHSLDSENAADKTVYRVSKGG
HITIDLYYKKNLTLTALDKSKVYDGTALSSSGLSDLNPSYSSELKTGDKLVAISYDGSQTNAGS
SATTPKLANISNASGADRTNFYHITGGRDLVL

Theoretical pl/Mw: 6.07 / 58708.25

The polynucleotide sequence (SEQ ID NO: 8) of the whole gene coding for Ssu05_0272 is:

gtgggaaaatgtataaatctatattttaagaaggagccaattatgagcaagcagaaagtt
gtgtccagtttattattaagcacagtcgtattaggggggattagcctttttattcaacgaca
acggttaaagcagaatcgctagaaccagatgttacatcagttaatgcaagcgatgctact
aataaaccggtagtaccaaatgaagagggaggcgactttttagctgaagaggagcttgaa
gatgatgacactttggaagaagaattaaatgagaaagccgaagaggttactgagccatct
tcacctgaagcacttctccaacctcgtgcgatgatgagtgattctgaaactagtggtatg
gaagaaattccgatgaatgatgaaccttcagataatacggaagaaaaggtagagaagcaa
cagtcgccattaattcaaacctcaaatgcagattataaaagtggtaaagatcaagagaaa
cttaggacatcagtatctataaatctgttgaaggcggaagaaggtcagattcagtggaaa
gtaacttttgatacaagcgaatggtcttttaatgttaaacatgggggtgtttattttatt
cttccaaatggtttggacttaacaaagattgttgataataatcaacatgatataacagcc
tccttccctacggatattaatgattacagaaattctggtcaagaaaaatatcgtttcttt
agtagtaaacaaggtcttgacaacgaaaatggttttaattctcaatggaactggtctgct
ggtcaagctaatcctagtgaaacagtaaacagttggaaatcaggcaatcgtttgtctaag
atttatttcataaatcaaataacagatactaccgaattgacctatactttaactgctaag
gtaactgaaccaaatcagcaatcattccctcttctggcagtgatgaagagctttacgtat
acaaactctaagagtacagaggttacttcgctaggtgcacgtgagattacccttgagaaa
gaaaaaactctaccacctaaggagaatccaaaaccagagccagaagctccaaaaccagac
gctccacaagcgccatcagcaccggaatctccaacggaagaacctaagaaggaagacgct
ccagcgccatcgactccggaaaaacaaccagaagtaccggaatcaccaaacccagagaca
ccagacgcgccatcgactccaaaagatgagccgcaagttccatcaatcccagaggagcaa
ccaaaagagactccagcgccagaagaacctaagaaggaagatactccgcaaacaccacaa
gcgccatcaactcctaaagaggaagcaccgaaggaagaagttccaacaccaccggctcca
tcagtaccagaagagcaaccaaaagaaactccaacaccagaagttccaaaacaagaagat
gttcaaccggaagcgccaaaatctgataaagtagaatctgacaaacaaatgccagaaact
aagaaaccagatatgaagcaaccgaaggcagacgacatgcctaaggaacaaaagccgaaa
gccgacgagccaaaggcagagcaaccacaaatggacaaaccacaaatggaagctcctaag
aaagattcggaagcgccaaaatctgataaagtagaaactgacaagcaattgccagaaact
aagcaaccagatatgaagcaaccgaaggcagacgacatgcctaaggaacaaaaaccgaaa
gccgacgagccaaaggcagagcaaccacaaatggacaaaccacaaatggaagctcctaag
aaagattcggaagcaccaaaatctgataaagtagaaactgacaaaccaatgccagaaact
aaacaaccagatatgaagcaaccgaaggcagataaaccagaagcagagaaagctcaaatg
ccacggacagagggtatgaagcctgaatctaaggcttcaatgatgccaaaagcagaggca
ccaaaagcaactttgccaaatacaggcgaagcaagtagcgcaataggctggctaggtggt
gctttagccactcttgccacaggcttgtatctattcaaaaacaaaaaagaagaatag

The amino acid sequence (SEQ ID NO: 9) coded by the above gene is:

MGKCINLYFKKEPIMSKQKVVSSLLLSTVVLGGLAFYSTTTVKAESLEPDVTSVNASDATNKPVVPNEEG

GDFLAEEELEDDDTLEEELNEKAEEVTEPSSPEALLQPRAMMSDSETSGMEEIPMNDEPSDNTEEKVEKQ

QSPLIQTSNADYKSGKDQEKLRTSVSINLLKAEEGQIQWKVTFDTSEWSFNVKHGGVYFILPNGLDLTKI

VDNNQHDITASFPTDINDYRNSGQEKYRFFSSKQGLDNENGFNSQWNWSAGQANPSETVNSWKSGNRLSK

IYFINQITDTTELTYTLTAKVTEPNQQSFPLLAVMKSFTYTNSKSTEVTSLGAREITLEKEKTLPPKENP

KPEPEAPKPDAPQAPSAPESPTEEPKKEDAPAPSTPEKQPEVPESPNPETPDAPSTPKDEPQVPSIPEEQ

PKETPAPEEPKKEDTPQTPQAPSTPKEEAPKEEVPTPPAPSVPEEQPKETPTPEVPKQEDVQPEAPKSDK

VESDKQMPETKKPDMKQPKADDMPKEQKPKADEPKAEQPQMDKPQMEAPKKDSEAPKSDKVETDKQLPET

KQPDMKQPKADDMPKEQKPKADEPKAEQPQMDKPQMEAPKKDSEAPKSDKVETDKPMPETKQPDMKQPKA

DKPEAEKAQMPRTEGMKPESKASMMPKAEAPKATLPNTGEASSAIGWLGGALATLATGLYLFKNKKEE

One fragment of the above coding gene was cloned. The nucleotide sequence (SEQ ID NO: 10) corresponding this fragment is:

GGGATCCGGGGTCAAGCTAATCCTAGTGAAACAGTAAACAGTTGGAAATCAGGCAATCGTTTGTCT
AAGATTTATTTCATAAATCAAATAACAGATACTACCGAATTGACCTATACTTTAACTGCTAAGGTA
ACTGAACCAAATCAGCAATCATTCCCTCTTCTGGCAGTGATGAAGAGCTTTACGTATACAAACTCT
AAGAGTACAGAGGTTACTTCGCTAGGTGCACGTGAGATTACCCTTGAGAAAGAAAAAACTCTACCA
CCTAAGGAGAATCCAAAACCAGAGCCAGAAGCTCCAAAACCAGACGCTCCACAAGCGCCATCAGCA
CCGGAATCTCCAACGGAAGAACCTAAGAAGGAAGACGCTCCAGCGCCATCGACTCCGGAAAAACAA
CCAGAAGTACCGGAATCACCAAACCCAGAGACACCAGACGCGCCATCGACTCCAAAAGATGAGCCG
CAAGTTCCATCAATCCCAGAGGAGCAACCAAAAGAGACTCCAGCGCCAGAAGAACCTAAGAAGGAA
GATACTCCGCAAACACCACAAGCGCCATCAACTCCTAAAGAGGAAGCACCGAAGGAAGAAGTTCCA
ACACCACCGGCTCCATCAGTACCAGAAGAGCAACCAAAAGAAACTCCAACACCAGAAGTTCCAAAA
CAAGAAGATGTTCAACCGGAAGCGCCAAAATCTGATAAAGTAGAATCTGACAAACAAATGCCAGAA
ACTAAGAAACCAGATATGAAGCAACCGAAGGCAGACGACATGCCTAAGGAACAAAAGCCGAAAGCC
GACGAGCCAAAGGCAGAGCAACCACAAATGGACAAACCACAAATGGAAGCTCCTAAGAAAGATTCG
GAAGCGCCAAAATCTGATAAAGTAGAAACTGACAAGCAATTGCCAGAAACTAAGCAACCAGATATG
AAGCAACCGAAGGCAGACGACATGCCTAAGGAACAAAAACCGAAAGCCGACGAGCCAAAGGCAGAG
CAACCACAAATGGACAAACCACAAATGGAAGCTCCTAAGAAAGATTCGGAAGCACCAAAATCTGAT
AAAGTAGAAACTGACAAACCAATGCCAGAAACTAAACAACCAGATATGAAGCAACCGAAGGCAGAT
AAACCAGAAGCAGAGAAAGCTCAAATGCCACGGACAGAGGGTATGAAGCCTGAATCTAAGGCTTCA
ATGATGCCAAAAGCAGAGGCACCAAAAGCAACTTTGCCAAATACAGGCGAAGCAAGTAGCGCAATA
GGCTGGCTAGGTGGTGCTTTAGCCACTCTTGCCACAGGCTTGTATCTACCCCGGG

The amino acid sequence coded by the above nucleotide sequence (SEQ ID NO: 11) is:

MKLKVTVNGTAYDVDVDVDKSHENPMGTILFGGGTGGAPAPAAGGAGAGKAGEGEIPAPLAGTVSK
ILVKEGDTVKAGQTVLVLEAMKMETEINAPTDGKVEKVLVKERDAVQGGQGLIKIGDLELIEGREA
SAGIRGQANPSETVNSWKSGNRLSKIYFINQITDTTELTYTLTAKVTEPNQQSFPLLAVMKSFTYT
NSKSTEVTSLGAREITLEKEKTLPPKENPKPEPEAPKPDAPQAPSAPESPTEEPKKEDAPAPSTPE
KQPEVPESPNPETPDAPSTPKDEPQVPSIPEEQPKETPAPEEPKKEDTPQTPQAPSTPKEEAPKEE
VPTPPAPSVPEEQPKETPTPEVPKQEDVQPEAPKSDKVESDKQMPETKKPDMKQPKADDMPKEQKP
KADEPKAEQPQMDKPQMEAPKKDSEAPKSDKVETDKQLPETKQPDMKQPKADDMPKEQKPKADEPK
AEQPQMDKPQMEAPKKDSEAPKSDKVETDKPMPETKQPDMKQPKADKPEAEKAQMPRTEGMKPESK
ASMMPKAEAPKATLPNTGEASSAIGWLGGALATLATGLYLPRGGRDLVL

Theoretical pl/Mw: 4.86 / 62064.80.

Another object of the present invention are the proteins Ssu05_0473 and Ssu05_0272 or immunogenic derivatives thereof, in particular the fragment aa853-aa1252 of protein Ssu05_0473, for use as medicament against Streptococcus suis colonization or infection.

Said proteins and immunogenic derivatives can be used either singularly or in combination with each other as medicament.

The present invention also refers to a vaccine at least one component selected from the group consisting of protein Ssu05_0473, Ssu05_0272 and immunogenic derivatives thereof, in particular the fragment aa853-aa1252 of protein Ssu05_0473, optionally comprising an adjuvant. It is intended that the vaccine according to the present invention can comprise as active ingredient the protein Ssu05_0473 or the protein Ssu05_0272 or an immunogenic derivative thereof or any combination of said active ingredients.

Another object of the present invention is a method for the diagnosis of bacterial infections comprising the following steps:
a. Contacting an isolated biological sample of a subject with proteins Ssu05_0473 and Ssu05_0272 and/or at least one immunogenic derivative thereof, in particular the fragment aa853-aa1252 of protein Ssu05_0473;
b. detecting antigen-antibody complex formation.

The present invention also refers to a method for the diagnosis of bacterial infections comprising the following steps:
a. contacting an isolated biological sample of a subject with an antibody recognizing the protein Ssu05_0473 and Ssu05_0272 and/or at least one immunogenic derivative thereof, in particular the fragment aa853-aa1252 of protein Ssu05_0473;
b. detecting antigen-antibody complex formation.

Another object of the present invention is a kit for the diagnosis of Streptococcus suis infections containing the proteins Ssu05_0473 and Ssu05_0272 and/or at least one immunogenic derivatives thereof.

These and other objects of the present invention will now be illustrated in detail also by means of example.

### Detailed description of the invention

The present invention provides the proteins Ssu05_0473 and Ssu05_0272 or their antigen fragments for use as medicament, in particular against Streptococcus suis colonization or infection.

According to the present invention, the same use is provided for immunogenic fragments of the protein Ssu05_0473, in particular the fragment aa853-aa1252.

Within the terms of the present invention, immunogenic fragment is an antigen fragment that substantially retains the capacity of eliciting an immune response, such as a protective response to a S. suis strain challenge when said fragment is administered to an animal.

In a preferred embodiment, the present invention provides a vaccine comprising the proteins Ssu05_0473 or Ssu05_0272 or both and/or at least one immunogenic fragment thereof, in particular the fragment aa853-aa1252 of protein Ssu05_0473.

In another preferred embodiment, the vaccine according to the present invention further comprises at least an adjuvant.

As used herein, the term "adjuvant" means a substance added to the composition of the invention to increase the composition's immunogenicity. The mechanism of how an adjuvant operates is not entirely known. Some adjuvants are believed to enhance the immune response (humoral and/or cellular response) by slowly releasing the antigen, while other adjuvants are strongly immunogenic in their own right and are believed to function synergistically. Known adjuvants include, but are not limited to, oil and water emulsions (for example, complete Freund's adjuvant and incomplete Freund's adjuvant), Corynebacterium parvum, Quil A, cytokines such as IL-12, Emulsigen-Plus(R), Bacillus Calmette Guerin, aluminum hydroxide, glucan, dextran sulfate, iron oxide, sodium alginate, Bacto Adjuvant, certain synthetic polymers such as poly amino acids and co-polymers of amino acids, saponin, paraffin oil, and muramyl dipeptide. Adjuvants also encompass genetic adjuvants such as immunomodulatory molecules encoded in a co-inoculated DNA, or as CpG oligonucleotides. The coinoculated DNA can be in the same plasmid construct as the plasmid immunogen or in a separate DNA vector.

Alternatively, the vaccine composition of the invention can comprise an antibody and/or a polynucleotide and/or an expression vector of the invention.

According to this further embodiment of the present invention, the antibody can be a purified polyclonal or monoclonal antibody that specifically binds to the S. suis proteins as defined above.

The antibodies used in the present invention may be prepared by a variety of methods known to one skilled in the art. For example, the protein of the invention may be administered to an animal in order to induce the production of polyclonal antibodies. Alternatively, antibodies used as described herein may be monoclonal antibodies, which are prepared using known hybridoma technologies (see, e.g., Hammerling et al, In Monoclonal Antibodies and T-Cell Hybridomas, Elsevier, NY, 1981 ; Charland, N., M. Jacques, S. lacouture and M. Gottschalk. 1997. Characterization and protective activity of a monoclonal antibody against a capsular epitope shared by Streptococcus suis serotypes 1, 2 and Vz. Microbiology 143 (Pt 11 ): 3607-14).

With respect to antibodies used in the present invention, the term "specifically binds to" refers to antibodies that bind with a relatively high affinity to one or more epitopes of the proteins Ssu05_473 and Ssu05_0272 of the invention. As used herein, the term "relatively high affinity" means a binding affinity between the antibody and said protein of at least 10⁶ M⁻¹, and preferably of at least about 10⁷ M⁻¹ and even more preferably 10⁸ M⁻¹to 10¹⁰ M⁻¹. Determination of such affinity is preferably conducted under standard competitive binding immunoassay conditions which are common knowledge to one skilled in the art.

The present invention also concerns an isolated polynucleotide encoding the proteins of the invention for the same uses as the proteins itself. Preferably, the isolated polynucleotide of the invention comprises a nucleotide sequence substantially identical to the sequence of Ssu05_0473 and Ssu05_0272 or their respective functional fragments thereof.

By "substantially identical" when referring to a nucleic acid sequence, it will be understood that the polynucleotide of the invention preferably has a nucleic acid sequence which is at least 65% identical, more particularly 80% identical and even more particularly 95% identical to part or all of the encoding sequence known.

A "functional fragment", as is generally understood and used herein, refers to a nucleic acid sequence that encodes for a functional biological activity that is substantially similar to the biological activity of the whole nucleic acid sequence. In other words, and within the context of the present invention, it preferably refers to a nucleic acid or fragment(s) thereof that substantially retains the capacity of encoding a polypeptide/protein which elicits an immune response, and more preferably a protective response, to a Streptococcus suis strain challenge when administered to an animal.

In another embodiment, the invention is further directed to vector (e.g., cloning or expression vector) comprising a polynucleotide of the invention as defined above.

As used herein, the term "vector" refers to a polynucleotide construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, or a "viral vector" which is designed to result in the production of a recombinant virus or virus-like particle, or "shuttle vectors", which comprises the attributes of more than one type of vectors.

A number of vectors suitable for stable transfection of cells and bacteria are available (e.g., plasmids, adenoviruses, baculoviruses, yeast baculoviruses, plant viruses, adenoassociated viruses, retroviruses, Herpes Simplex Viruses, Alphaviruses, Lentiviruses), as are methods for constructing such cell lines. It will be understood that the present invention encompasses any type of vector comprising any of the polynucleotide molecule of the invention.

The proteins Ssu05_473 and Ssu05_0272, their fragments, polynucleotides encoding same and antibodies of the invention may be used in many ways in the treatment and/or prevention of Streptococcus suis-associated diseases or infection caused by S. suis.

For instance, and according to an aspect of the invention, the proteins of the invention may be used as immunogens for the production of specific antibodies for the treatment and/or prevention of Streptococcus suis infection. Suitable antibodies may be determined using appropriate screening methods, for example by measuring the ability of a particular antibody to passively protect against Streptococcus suis infection in a test model. Examples of an animal model are the mouse and pig models described in the examples herein.

According to another aspect, the polynucleotides encoding polypeptides of the invention or derivatives thereof may be used in a DNA immunization method.

That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

The use of a polynucleotide of the invention in genetic immunization will preferably employ a suitable delivery method or system such as direct injection of plasmid DNA into muscles (Wolf et al. H M G (1992) 1 : 363, Turnes et al., Vaccine (1999), 17 : 2089, Le et al. , Vaccine (2000) 18 : 1893, Alves et al. , Vaccine (2001)19 : 788), injection of plasmid DNA with or without adjuvants (Ulmer et al. , Vaccine (1999) 18: 18, MacLaughlin et al. , J. Control Release (1998) 56: 259, Hartikka et al. , Gene Ther. (2000) 7: 1171-82, Benvenisty and Reshef, PNAS USA (1986) 83: 9551 , Singh et al. , PNAS USA (2000) 97: 811), targeting cells by delivery of DNA complexed with specific carriers (Wa et al. , J Biol Chem (1989) 264: 16985, Chaplin et al., Infect. Immun. (1999) 67:6434), injection of plasmid complexed or encapsulated in various forms of liposomes (Ishii et al. , AIDS Research and Human Retroviruses (1997) 13: 142, Perrie et al., Vaccine (2001) 19:3301), administration of DNA with different methods of bombardment (Tang et al., Nature (1992) 356: 152, Eisenbraun et al. , DNA Cell Biol (1993) 12: 791 , Chen et al. , Vaccine (2001 ) 19:2908), and administration of DNA with lived vectors (Tubulekas et al. , Gene (1997) 190: 191 , Pushko et al., Virology (1997) 239: 389, Spreng et al. FEMS (2000) 27: 299, Dietrich et al., Vaccine (2001 ) 19: 2506).

A further aspect of the invention is the use of the antibodies directed to the polypeptides of the invention for passive immunization. One could use the antibodies described in the present application.

Therefore, according to a general aspect of the invention, the term "immunogenic derivative" comprises the above explained immunogenic fragments of the protein of the present invention, the antibodies, polynucleotides and vectors.

Yet, a further embodiment of the present invention is to provide a method for treating and/or preventing a Streptococcus suis-associated disease or infection in an animal. The method of the invention comprises the step of administering to the animal a composition according to the invention.

Further agents can be added to the composition of the invention. For instance, the composition of the invention may also comprise agents such as drugs, immunostimulants (such as [alpha]-interferon, [beta]-interferon, [gamma]-interferon, granulocyte macrophage colony stimulator factor (GM-CSF), macrophage colony stimulator factor (M-CSF), and interleukin 2 (IL-2)), antioxidants, surfactants, flavoring agents, volatile oils, buffering agents, dispersants, propellants, and preservatives. For preparing such compositions, methods well known in the art may be used. The amount of the components or the elements of the composition of the invention is preferably a therapeutically effective amount. A therapeutically effective amount of the contemplated component is the amount necessary to allow the same to perform their immunological role without causing overly negative effects in the host to which the composition is administered. The exact amount of the components to be used and the composition to be administered will vary according to factors such as the type of condition being treated, the type and age of the animal to be treated, the mode of administration, as well as the other ingredients in the composition.

The composition of the invention may be given to an animal through various routes of administration. For instance, the composition may be administered in the form of sterile injectable preparations, such as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parenterally-acceptable diluents or solvents. They may be given parenterally, for example intravenously, intramuscularly or subcutaneously by injection, by infusion or per os. Suitable dosages will vary, depending upon factors such as the amount of each of the components in the composition, the desired effect (short or long term), the route of administration, the age and the weight of the animal to be treated. Any other methods well known in the art may be used for administering the composition of the invention.

The proteins Ssu05_0473 and Ssu05_0272, polynucleotides encoding same and antibodies of the invention may also be used in different ways in the detection and diagnosis of Streptococcus suis-associated diseases or infections caused by S. suis.

In this connection and in a further embodiment, the present invention provides a method for detecting the presence or absence of a Streptococcus suis strain in a sample, comprising the steps of:
a) contacting the sample with an antibody of the invention as defined above for a time and under conditions sufficient to form a complex; and
b) detecting the presence or absence of the complex formed in a).

As used herein, the term "sample" refers to a variety of sample types obtained from an animal and can be used in a diagnostic or detection assay. The definition encompasses blood and other liquid samples of biological origin, solid tissue samples such as a biopsy specimen or tissue culture or cells derived therefrom.

Yet, in another embodiment, the present invention provides a method for detecting the presence or absence of antibodies raised against a Streptococcus suis strain in a sample, comprising the steps of:
a) contacting the sample with a protein and/or a polypeptide and or an immunogenic derivative of the invention as defined above for a time and under conditions sufficient to form an immune complex; and
b) detecting the presence or absence of the immune complex formed in a).

One skilled in the art will recognize that this diagnostic test may take several forms, including an immunological test such as an enzyme-linked immunosorbent assay (ELISA) or a radioimmunoassay, essentially to determine whether antibodies specific for the protein are present in an organism.

The present invention further provides kits for use within any of the above diagnostic methods. Such kits typically comprise two or more components necessary for performing a diagnostic assay. Components may be compounds, reagents, containers and/or equipment. For example, one container within a kit may contain an antibody or fragment thereof that specifically binds to proteins Ssu05_0473 and Ssu05_0272 of the invention. One or more additional containers may enclose elements, such as reagents or buffers, to be used in the assay.

In this connection, the present invention also provides a diagnostic kit for the detection of the presence or absence of antibodies indicative of Streptococcus suis strain, comprising:
- a protein Ssu05_0473 and/or a protein Ssu05_0272 and/or an immunogenic derivative thereof according to the invention;
- a reagent to detect polypeptide-antibody immune complex;
- a biological reference sample lacking antibodies that immunologically bind with said polypeptide; and
- a comparison sample comprising antibodies which can specifically bind to said polypeptide; wherein said polypeptide, reagent, biological reference sample, and comparison sample are present in an amount sufficient to perform said detection.

Another diagnostic kit preferably contemplated is a kit for the detection of the presence or absence of polypeptides indicative of Streptococcus suis strain, comprising:
- an antibody according to the invention;
- a reagent to detect polypeptide-antibody immune complex;
- a biological reference sample polypeptides that immunologically binds with said antibody; and
- a comparison sample comprising polypeptides which can specifically bind to said peptide; wherein said antibody, reagent, biological reference sample, and comparison sample are present in an amount sufficient to perform said detection.

The following example further illustrates the invention.

### Example

### Material and Methods:

### Bacterial strains and growth.

Streptococcus suis serotype 2, strain 235/02, isolated from an infected pig in Córdoba, Spain in 2002, was grown in Todd-Hewitt broth supplemented with 0.5% yeast extract (THY) at 37 °C and 5% CO₂, until an OD₆₀₀ of 0.25 (mid-exponential phase) was reached.

### Surface digestion of live cells and viability assays.

One hundred ml of bacteria from mid-exponential growth phase (corresponding to approximately 10¹⁰ cells at OD₆₀₀ = 0.25) were harvested by centrifugation at 3,500 × g for 10 min at 4 °C, and washed three times with PBS. Cells were resuspended in 0.8 ml of incubation buffer consisting of PBS/30% sucrose (pH 7.4 for trypsin digestion and pH 6.0 for proteinase K digestion). Proteolytic reactions were carried out with trypsin (Promega) at 10 µg/ml or proteinase K (Sigma) at 5 µg/ml, for 20 min at 37 °C. Controls were carried out without adding any enzyme. The digestion mixtures were centrifuged at 3,500 × g for 10 min at 4 °C, and the supernatants (containing the peptides and large poplypeptides not fully digested) were filtered using 0.22-µm pore-size filters (Millipore). An aliquot of each digestion reaction was re-digested each one with the same enzyme and concentration, trypsin digestion for 2 h and proteinase-K digestion for 20 min. Protease reactions were stopped with formic acid at 0.1% final concentration. Before analysis, salts were removed by using commercial mini-cartridges HLB-Oasis (Waters) and then eluting the peptides with increasing concentrations of acetonitrile, according to manufacturer's instructions. Peptide fractions were concentrated with a vacuum concentrator (Eppendorf), and kept in low-binding tubes at -20 °C until further analysis. Viability of treated and non-treated bacteria with proteases was assayed by counting CFUs (colony-forming units) in THY plates containing 5% defibrinated sheep blood.

LC/MS/MS analysis.

All analyses were performed with a Surveyor HPLC System in tandem with a Finnigan LTQ mass spectrometer (Thermo Fisher Scientific, San Jose, USA) equipped with nanoelectrospray ionization interface (nESI). The separation column was 150 mm x 0.150 mm ProteoPep2 C18 (New Objective, USA) at a postsplit flow rate of 1 µl/min. For trapping of the digest a 5 mm x 0.3 mm precolumn Zorbax 300 SB-C18 (Agilent Technologies, Germany) was used. One fourth of the total sample volume, corresponding to 5 µl, was trapped at a flow rate of 10 µl/min for 10 minutes and 5% acetonitrile/0.1% formic acid. After that, the trapping column was switched on-line with the separation column and the gradient was started. Peptides were eluted with a 60-min gradient of 5-40% of acetonitrile/0.1% formic acid solution at a 250 nl/min flow rate. All separations were performed using a gradient of 5-40% solvent B for 60 minutes. MS data (Full Scan) were acquired in the positive ion mode over the 400-1500 m/z range. MS/MS data were acquired in dependent scan mode, selecting automatically the five most intense ions for fragmentation, with dynamic exclusion set to on. In all cases, a nESI spray voltage of 1.9 kV was used.

### Database searching and protein identification.

Search and identification of peptides were performed using in batch mode the raw MS/MS data with a licensed version of MASCOT, in a non-redundant local database containing the 2,185 proteins derived from the complete genome sequence of Streptococcus suis strain 05ZYH33 (RefSeq NC_009442 downloaded from ftp://ftp.ncbi.nih.gov/genomes/Bacteria/Streptococcus_suis_05ZYH33, NCBInr version 20071013). The MASCOT search parameters were: (i) species, Streptococcus suis strain 05ZYH33; (ii) allowed number of missed cleavages (only for trypsin digestion), 4; (iii) variable post-translational modifications, methionine oxidation, and deamidation of asparagine and glutamine residues; (iv) peptide mass tolerance, ±500 p.p.m.; (v) fragment mass tolerance, ±0.6 Da and (vi) peptide charge, from +1 to +4. The score thresholds for acceptance of protein identifications from at least one peptide were set by MASCOT as 19 for trypsin cleavage and 34 for proteinase K digestion. All spectra corresponding to positive identifications or near the thresholds were manually inspected.

### Bioinformatic analysis of protein sequences.

Computational predictions of subcellular localization were carried out using the web-based algorithm PSORTb v 2.0 (http://www.psort.org/psortb). Feature-based algorithms were also used to contrast PSORTb predictions, especially when it returned an "unknown" output: TMHMM 2.0 (http://www.cbs.dtu.dk/services/TMHMM-2.0) for searching transmembrane helices; SignalP 3.0 (http://www.cbs.dtu.dk/services/SignaIP) for type-I signal peptides: those proteins containing only a cleavable type-I signal peptide as featured sequence were classed as secreted; LipoP (http://www.cbs.dtu.dk/services/LipoP) for identifying type-II signal peptides, which are characteristic of lipoproteins. Similarity searches were carried out using the BLAST suite (http://www.ncbi.nlm.nih.gov/blast/Blast.cgi). Gene Ontology (GO) annotations were retrieved using the AmiGO browser (http://amigo.geneontology.org/cgi-bin/amigo/go.cgi). Additional information on protein families, motifs, predictions of subcellular localization and status of the protein was retrieved from UniProt Knowledgebase (http://www.uniprot.org).

### Expression and purification of Streptococcus suis proteins and preparation of immune sera

Cloning, expression and purification of recombinant proteins, and preparation of immune sera, were performed as follows. Briefly, the chromosomal DNA of strain 235/02 was used for gene amplification. PCR primers were designed so as to amplify genes or gene fragments without predicted signal peptide coding sequences. The sequences of the primers used for amplifying the fragment FA1 of Ssu05_0473 are:
FA1_Up CGCGGATCCGGGAGTCTGCTAGC (SEQ ID NO: 12) with restriction site (BamHI),
FA1_Low GAGTCATTCTGCGGCCGCCAAAGGTTTTCCG (SEQ ID NO: 13) with restriction site (NotI).

The sequences of the primers used for amplifying the fragment FA3 of Ssu05_0473 are:
FA3_up CGCGGATCCGGAATAAGATGCCTG (SEQ ID NO: 14) with BamHI restriction site,
FA3_low GAGTCATTCTGCGGCCGCCGGTTATGTGATAG (SEQ ID NO: 15) with NotI restriction site.

The sequences of the primers used for amplifying the fragment of Ssu05_0272 are:
up CGCGGATCCGGGGTCAAGCTAATC (SEQ ID NO: 16) with restriction site (BamHI),
Low_Sma TCCGGACCCGGGGTAGATACAAGCC (SEQ ID NO: 17) with restriction site (Smal).

PCR products were introduced into a commercial vector [PinPoint Xa1] so as to generate biotin-tagged recombinant proteins (PinPoint Xa1 Protein Purification System, Promega). E.coli JM109 cells were used as the recipient. Recombinant proteins were affinity-purified with SoftLink Soft Relase Avidin Resin (Promega), and 3 doses of 50 µg each were used to immunize groups of adult CD1 mice (Charles River, 10 mice per group) at days 0, 21 and 42. Mice were bled 2 weeks after the third immunization. The sera of each group were pooled and stored at -80°C.

### FACS analysis of surface proteins

Bacteria were grown in THB until mid-exponential phase was reached, then washed twice with PBS, fixed in 2% paraformaldehyde, suspended in NCS (Newborn Calf Serum, Sigma), incubated for 20 min at RT and dispensed in a 96-well plate (20µl/well). Eighty µl of pre-immune or immune mouse sera, diluted in PBS containing 0.1 % BSA, were added to the bacterial suspension to a final dilution of 1:80 and incubation was performed on ice for 30 min. After washing twice with 0.1 %BSA in PBS, bacteria were incubated on ice for 30 min in 10 µl of Goat Anti-Mouse IgG, F(ab')₂ fragment-specific-R-Phycoerythrin-conjugated diluted 1:50 in PBS containing 0.1% BSA-20% NCS. Following incubation, bacteria were washed with PBS-0.1% BSA, suspended in 200µl PBS and analyzed using a FACS Calibur cytometer (Becton Dikinson, Mountain View, CA, USA) and Cell Quest Software (Becton Dikinson).

### Screening of protective antigens in the mouse model

Eight-week-old, specific pathogen-free CD1 mice (Charles River, 10 mice/group) were immunized with 50 µg of recombinant proteins administered intraperitoneally at 0, 21 and 42 days with complete Freund adjuvant (CFA) (priming dose) and incomplete Freund adjuvant (IFA) (booster doses). Positive controls consisted of immunization with Sao-like protein. A negative control group consisted of animals immunized with a biotin-tagged irrelevant protein (chloramphenicol acetyl transferase, CAT-BIO). Blood samples were collected before the first (pre-immune sera) and after the third immunizations (immune sera). Immunized mice were challenged intravenously (i.v.) with 100 µl of THB containing 2 ×10⁷ colony forming units (CFUs) of S. suis strain 235/02 grown in THB broth at mid-exponential phase. Protection was determined from the bacterial burden in the kidneys at 4 days after intravenous infection, by counting the CFUs after plating in TH plates supplemented with 0.5% yeast extract and 5% defibrinated sheep blood.

Results:

Table 1 shows the list of identified proteins by the previously described proteomics approach when applied to Streptococcus suis strain 235/02, serotype 2:

**Table 1**

| Surface proteins identified by LC/MS/MS after protease treatment of cultured live cells of Streptococcus suis serotype 2, strain 235/02. The table reports: 1) the accession number of the genes encoding the identified proteins, 2) the predicted cellular localization of the proteins, 3) the algorithms used for such predictions. | | |
|---|---|---|
| Gene locus, | Predicted subcellular | Prediction algorithm |
| Annotated protein function | localization | |
| ssu05_0753, | Cell wall | PSORTb v 2.0 |
| MRP | | |
| ssu05_1982, | Cell wall | PSORTb v 2.0 |
| Subtilisin-like serine protease | | |
| ssu05_1968, | Cell wall | PSORTb v 2.0 |
| DNA nuclease | | |
| ssu05_0196, | Cell wall | PSORTb v 2.0 |
| hypothetical protein | | |
| SSU05_0196 | | |
| ssu05_1311, | Cell wall | PSORTb v 2.0 |
| hypothetical protein | | |
| SSU05_1311 | | |
| ssu05_0965, | Cell wall | PSORTb v 2.0 |
| agglutinin receptor | | |
| ssu05_2064, | Cell wall | PSORTb v 2.0 |
| Type II secretory pathway, | | |
| pullulanase PulA and related | | |
| glycosidases | | |
| ssu05_1371, | Cell wall | PSORTb v 2.0 |
| Ribonucleases G and E | | |
| ssu05_1295, | Cell wall | PSORTb v 2.0 |
| hypothetical protein | | |
| SSU05_1295 | | |
| ssu05_0214, | Cell wall | PSORTb v 2.0 |
| ABC-type xylose transport | | |
| system, periplasmic | | |
| component | | |
| ssu05_1663, | Cell wall | PSORTb v 2.0 |
| Methyl-accepting chemotaxis | | |
| protein | | |
| ssu05_0473, | Cell wall | PSORTb v 2.0 |
| Ribonucleases G and E | | |
| Ssu05_0272, | Cell wall | PSORTb v 2.0 |
| Translation initiation factor 2 | | |
| (IF-2; GTPase) | | |
| ssu05_0700, | Lipoprotein | LipoP |
| ATPase (PiIT family) | | |
| ssu05_1083, | Lipoprotein | LipoP |
| Uncharacterized ABC-type | | |
| transport system, periplasmic | | |
| component/surface lipoprotein | | |
| ssu05_2133, | Lipoprotein | LipoP |
| ABC transporter substrate- | | |
| binding protein - | | |
| maltose/maltodextrin | | |
| ssu05_0513, | Membrane | TMHMM |
| Membrane-fusion protein | | |
| ssu05_1022, | Membrane | TMHMM |
| hypothetical protein | | |
| SSU05_1022 | | |
| ssu05_1635, | Membrane | TMHMM |
| Predicted xylanase/chitin | | |
| deacetylase | | |
| ssu05_1354, | Membrane | TMHMM |
| Cell division protein | | |
| Ftsl/penicillin-binding protein 2 | | |
| ssu05_1509, | Membrane | TMHMM |
| Negative regulator of septation | | |
| ring formation | | |
| ssu05_2173, | Membrane | TMHMM |
| LysM repeat protein | | |
| ssu05_1579, | Membrane | PSORTb v 2.0, TMHMM |
| Ammonia permease | | |
| ssu05_1292, | Membrane | PSORTb v 2.0, TMHMM |
| Phosphoglycerol transferase | | |
| and related proteins, alkaline | | |
| phosphatase superfamily | | |
| ssu05_1380, | Membrane | TMHMM |
| ABC-type antimicrobial peptide | | |
| transport system, permease | | |
| component | | |
| ssu05_1282, | Membrane | PSORTb v 2.0, TMHMM |
| Predicted membrane protein | | |
| ssu05_0332, | Secreted | SignalP |
| hypothetical protein | | |
| SSU05_0332 | | |
| ssu05_0811, | Secreted | SignalP |
| Subtilisin-like serine protease | | |
| ssu05_1682, | Secreted | SignalP |
| extracellular serine protease | | |

For three identified proteins, coded by the loci ssu05_1371, ssu05_0473 (both annotated as "ribonucleases G and E") and ssu05_0272 (annotated as "Translation initiation factor 2 (IF-2; GTPase)), the assigned functions were not in agreement with their primary sequences. When examining the sequences at the amino acid level, they showed the canonical architecture of the cell-wall attached proteins.

Ribonucleases G and E are a family of endonucleases involved in RNA processing: their cellular localization is, therefore, cytoplasmic. They are mainly present in Gram-negative bacteria, and rarely detected in Gram-positive organisms. In fact, Ssu05_1371 is not similar to any of the proteins annotated as ribonucleases G and E in the databases. However, similarity (83% identity) was found to protein Sao from Streptococcus suis (GenBank accession number AY864331, and named "surface protein SP1" in the not yet fully annotated strain 89/1591), which has been shown to be surface-located and also to protect immunised animals against infection. Moreover, Ssu05_1371 was highly similar to other streptococcal proteins that have characteristic functions attributed to surface proteins, as binding to the extracellular matrix (ECM) of the host cells. These proteins are known generally as adhesins. Adhesins mediate adherence to host cells or tissues during the first steps of infection.

The locus ssu05_0473 codes for a large protein of 1603 amino acids, showing the structure of a cell-wall protein with the LPXTG (SEQ ID NO: 1) motif. This locus is in a region that is analogous to the pilus island 2b (PI-2b) from Streptococcus agalactiae COH1, which has recently been found in the draft genome of S. suis P1/7 (Fittipaldi et al, Appl Environ Microbiol 2007). Pili are filamentous structures that, in Gram-positive organisms, serve to adhere and invade host cells. Protein Ssu05_0473 was not identified by trypsin digestion and this is reminiscent of the S. pyogenes pilin proteins which were previously named "T antigens" (for "trypsin resistant"). The identification of protein Ssu05_0473 was achieved by two peptides after proteinase K treatment. Protein Ssu05_0272 has been also wrongly annotated as "Translation initiation factor 2 (IF-2; GTP-ase)" (translation initiation factors are cytoplasmic proteins), 698 amino acids long and also containing the cell-wall sorting signal. No strong similarity was found to other proteins. However, slight similarity was found to proteins from other Gram-positive organisms with adhesin function, or even to a collagen-like protein from Streptococcus equis subsp. zooepidemicus (locus tag: sez_0729) or a collagen-like surface protein from Streptococcus pyogenes. Pattern and profile searches (InterPro, Pfam, PROSITE) did not reveal other domains apart from Gram-positive, LPXTG-dependent (SEQ ID NO: 1) anchoring to the cell wall. These three proteins were used in a protection assay in which animals were immunised intraperitoneally with said proteins (boosts at days 0, 14 and 28), and then challenged intravenously (at day 49) with S. suis strain 235/02. Protective capacity was measured by means of the decrease in CFUs recovered from the kidneys of infected animals: the lower the number of CFUs, the more the protein protected. Protein Ssu05_1371, previously identified as Sao and demonstrated to protect immunised mice (Li et al, 2006), was used as positive control. Two recombinant fragments of protein Ssu05_0473 (aa51-aa451 and aa853-aa1252) were tested. Table 2 shows the mean values of the CFUs/ml from kidneys after 96-hours challenge. Fragment aa853-1252 of Ssu05_0473 and protein Ssu05_1371 showed a clear protective activity with respect to the control (CAT-bio), and the protection capacity was especially high for animals immunised with Ssu05_0272.

**Table 2**

| Immunogen | Kidney CFU/ml |
|---|---|
| | (96 h after challenge) |
| K- (CAT-bio): | 9x10⁴ (DS=1x10⁵ ) |
| Chloramphenicol acetyltransferase -biotine | |
| SSU_0473/FA1 (400aa;a51-a451) | 1x10⁵ (DS=1,9x10⁵ ) |
| SSU_0473/FA3 ( 399 aa;a853-a1252) | 1x10⁴ (DS=3x10⁴ ) |
| SSU_1371 ( 551aa;a50-a601) | 3x10⁴ (DS=1x10⁵ ) |
| SSU_0272 | 5x10³ (DS=6x10³ ) |

Table 3 shows the percentage of protected animals for each immunogen (ten animals were used per group). The higher percentages were obtained for the fragment aa853-aa1252 of Ssu05_0473 and for protein Ssu05_1371.

**Table 3**

| Immunogen | Number of protected mice/total(%) |
|---|---|
| K- (CAT-bio): | 1/10(10%) |
| Chloramphenicol acetyltransferase -biotine | |
| SSU_0473 /FA1 (400aa;a51-a451) | 2/10(20%) |
| SSU_0473/FA3 ( 399 aa;a853-a1252) | 7/10(70%) |
| SSU_1371 ( 551aa;a50-a601) | 8/10(80%) |
| SSU_0272 | 3/10 (30%) |

### Industrial Application

The present invention relates to the medical field, in particular to the diagnosis and cure of infection of Streptococcus suis.

The present invention provides a vaccine against Streptococcus suis, useful to prevent and cure the infection caused by said pathogen, and a kit for the detection of Streptococcus suis infection with high reliability which can be fast and disposable.

## Claims

1. Protein Ssu05_0473 and Ssu05_0272 and immunogenic derivatives thereof for use as a medicament.

2. The immunogenic derivative of the protein Ssu05_0473 of claim 1, which is the fragment aa853-aa1252 for use as a medicament.

3. Protein Ssu05_0473 and Ssu05_0272 and immunogenic fragments thereof for use as medicament against Streptococcus suis colonization or infection.

4. The immunogenic derivative of the protein Ssu05_0473 of claim 3, which is the fragment aa853-aa1252 for use as a medicament against Streptococcus suis colonization or infection.

5. A vaccine comprising at least one component selected from the group consisting of protein Ssu05_0473, Ssu05_0272 and immunogenic derivatives thereof.

6. The vaccine of claim 5 wherein said immunogenic derivative is the fragment aa853-aa1252 of the protein Ssu05_0473.

7. The vaccine according to claim 5 or 6, further comprising at least an adjuvant.

8. Method for the diagnosis of bacterial infections comprising the following steps:
a. contacting an isolated biological sample of a subject with the protein of claim 1 and/or at least one immunogenic derivative thereof;
b. detecting antigen-antibody complex formation.

9. Method according to claim 8, wherein said derivative is the fragment aa853-aa1252 of the protein Ssu05_0473.

10. Method for the diagnosis of bacterial infections comprising the following steps:
a. contacting an isolated biological sample of a subject with an antibody recognizing the protein of claim 1 and/or at least one immunogenic derivative thereof;
b. detecting antigen-antibody complex formation.

11. Method according to claim 10, wherein said derivative is the fragment aa853-aa1252 of the protein Ssu05_0473.

12. Kit for the diagnosis of Streptococcus suis infections containing the protein and/or at least one immunogenic derivatives thereof of Claim 1.

13. Kit for the diagnosis of Streptococcus suis infections containing an antibody recognizing the protein and/or at least one immunogenic derivatives thereof of Claim 1.
